(19) Europäisches Patentamt
European Patent Office
Office européen des brevets

(11) Publication number: **0 065 874 A1**

(12) **EUROPEAN PATENT APPLICATION**

(21) Application number: 82302598.6

(22) Date of filing: 21.05.82

(51) Int. Cl.³: C 07 C 97/10
C 07 C 91/44, C 07 C 93/14
C 07 C 101/72, C 07 C 101/78
C 07 C 149/42, C 07 C 131/00
A 61 K 31/135, A 61 K 31/15
A 61 K 31/195, A 61 K 31/215

(30) Priority: 22.05.81 JP 77800/81
21.09.81 JP 147741/81
20.10.81 JP 166454/81

(43) Date of publication of application:
01.12.82 Bulletin 82/48

(84) Designated Contracting States:
AT BE CH DE FR GB IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Miyamoto, Tsumoru
No.1-40, Koaza Moriyama Aza Tono
Johyoh-shi Kyoto(JP)

(72) Inventor: Mohri, Tetsuya
No.14-3, Ohsumigaoka 4-chome Tanabe-cho
Tsuzuki-gun Kyoto(JP)

(72) Inventor: Shigeoka, Satoshi
No. 5-1, Hachiman-dai 3-chome
Neyagawa-shi Osaka(JP)

(72) Inventor: Itoh, Hiroyuki
No.16-20, Yamate-dai 3-chome
Ibaraki-shi Osaka(JP)

(72) Inventor: Hayashi, Masaki
No. 5-10, Nanpei-dai 4-chome
Takatsuki-shi Osaka(JP)

(74) Representative: Pearce, Anthony Richmond et al,
Marks & Clerk Alpha Tower ATV Centre
Birmingham B1 1TT(GB)

(54) **2-Aminophenol derivatives, methods for their preparation and composition containing the same.**

(57) 2-Aminophenol derivatives of general formula:

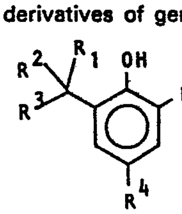

(1)

[wherein $R^1$ represents a straight or branched chain alkyl group containing from 1 to 6 carbon atom(s), a group represented by general formula:

$$-(CH_2)n-COOR^5 \qquad (II)$$

(wherein $R^5$ represents a hydrogen atom or a straight or branched chain alkyl group containing from 1 to 4 carbon atom(s) and n represents zero or an integer from 1 to 6) or a group represented by general formula:

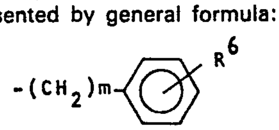

(111)

(wherein $R^6$ represents a hydrogen atom, a halogen atom, a straight or branched chain alkyl group containing from 1 to 4 carbon atom(s), a straight or branched chain alkoxyl group containing from 1 to 4 carbon atom(s), a carbamoyl group, an alkoxycarbonyl group containing from 2 to 6 carbon atoms, a carboxyl group, an amino group, a sulfamoyl group or a cyano group and m represents zero or an integer of 1 or 2.), $R^2$ represents a hydroxyl group, a straight or branched chain alkoxyl group containing from 1 to 4 carbon atom(s) or together with $R^3$ an oxo group or a hydroxyimino group. $R^3$ represents a hydrogen atom or together with $R^2$ an oxo group or a hydroxyimino group. $R^4$ represents a halogen atom, a straight or branched chain alkyl group containing from 1 to 6 carbon atom(s) or a phenylthio group. With the exclusion of a compound wherein $R^2$ and $R^3$ taken together represent an oxo group, $R^1$ represents a methyl group and $R^4$ represents a methyl group.]

./...

EP 0 065 874 A1

or their pharmaceutical acceptable acid addition salts which strongly inhibit 5-lipoxygenase specifically, or 5-lipoxygenase and cyclooxygenase at the same time, and are therefore useful as treating agents and preventing agents for diseases which are induced by leukotrienes e.g. allergic tracheal and bronchial diseases or allergic lung diseases, allergic shock and also various allergic inflammations which are induced by prostaglandins, and a process for their preparation and pharmaceutical compositions comprising them as active ingredients.

## SPECIFICATION

### 2-Aminophenol Derivatives, methods for their preparation and composition containing the same.

This invention is concerned to new 2-aminophenol derivatives which have an inhibitory activity on 5-lipoxygenase specifically or an inhibitory activity on 5-lipoxygenase and cyclooxygenase at the same time, a process for their preparation and a pharmaceutical composition containing them or it as active ingredient(s).

Up to now, several compounds which inhibit lipoxygenase have been known, that is to say, 5,8,11,14-eicosatetraynoic acid (i.e. EYTA). 5,8,11-eicosatrienioc acid, acetone phenylhydrazone (i.e. APH), phenidone (i.e. 1-phenyl-3-pyrazolidone), NDGA (nordihydroguaiaretic acid), BW-775C (3-amino-1-p-trifluoromethyl-pyrazole), 3-amino-1-p-chlorophenyl-2-pyrazoline, benoxaprofene.

These above compounds inhibit all lipoxygenases and other enzymes in the arachidonic cascade at the same time, or inhibit all liposygenases of 5-, 12- and 15-lipoxygenase [see Gendai Iryou 12, 1065 (1980)].

The compounds of the present invention are particularly superior in having selective inhibitory activity on 5-lipoxygenase of many lipoxygenases which react at the first stage of producing various kinds of leukotrienes from arachidonic acid in a living body and further not inhibiting other enzymes in the arachidonate cascade,or having inhibitory activity on 5-lipoxygenase and cyclooxygenase which reacts at the first step of producing prostaglandins from arachidonic acid similarly, at the same time.

This invention is concerned to new 2-aminophenol derivatives which have an inhibitory activity on 5-lipoxygenase or an inhibitory activity on 5-lipoxygenase and cyclooxygenase, a process for their preparation and a pharmaceutical composition containing them as active ingredients. More particularly, there are a group of compounds called leukotrienes, which are biosynthesized from arachidonic acid by a series of reactions in a living body and 5-lipoxygenase is related at the first step of this reaction, and similarly, prostaglandins are biosynthesized from arachidonic acid, and cyclooxygenase is related at the first step of the reaction. This invention is concerned to new 2-aminophenol derivatives which strongly inhibit 5-lipoxygenase,or 5-lipoxyganase and cyclooxygenase at the same time, and are therefore useful as treating agents and preventing agents for diseases which are induced by leukotrienes e.g. allergic tracheal and bronchial diseases or allergic lung diseases, allergic shock and also various allergic inflammations which are induced by prostaglandins, and a process for their preparation and pharmaceutical compositions comprising them as active ingredients.

In the study of prostaglandins (abbreviated as PG hereafter), many important discoveries have been made continuously in recent years. And so it was found a large change in the direction of the research and development of PG. In the compounds which have been newly found or newly confirmed their structure in PG family, it can be said that PG endoperoxides, (i.e. $PGG_2$ and $PGH_2$), thromboxane $A_2$ (abbreviated as $TXA_2$ hereafter), prostacyclin (i.e. $PGI_2$) and leukotrienes B, C, D and E (abbreviated as LTB, LTC, LTD and LTE, respectively, hereafter) etc. have

especially strong and unique biological activities.

All the compounds of PG family containing various PG known well in addition to the above compounds, are biosynthesized from the same mother compound, i.e. arachidonic acid and, therefore, the metabolic routes starting from arachidonic acid is called "Arachidonate cascade" as a whole. The detailed explanation of each route and the pharmacological character of each metabolite are described in Igaku no Ayumi, 114, 378 (1980), ibid, 114, 462 (1980), ibid, 114, 866 (1980), ibid, 114, 929 (1980), Gendai Iryo, 12, 909 (1980), ibid, 12, 1029 (1980), ibid, 12, 1065 (1980) and ibid, 12, 1105 (1980) etc.

The arachidonate cascade can be largely divided into two routes: one is the route that cyclooxygenase acts on arachidonic acid to convert into various PG e.g. prostaglandin $F_{2\alpha}$ (abbreviated $PGF_{2\alpha}$ hereafter), prostaglandin $E_2$ (abbreviated $PGE_2$ hereafter), $PGI_2$, $TXA_2$ through $PGG_2$ and the other is the route that lipoxygenase reacts on arachidonic acid to convert into hydroxyeicosatetraenoic acid (abbreviated as HETE hereafter) or leukotriene through hydroperoxyeicosatetraenoic acid (abbreviated HPETE hereafter).

Concerning the former route, it has been known well, so we do not describe here the details. See Makoto Katori et al, Prostaglandin (1978) published by Kohdan-sha.

Concerning the latter route, it has been known that various componds are produced according to the following scheme I.

Scheme I

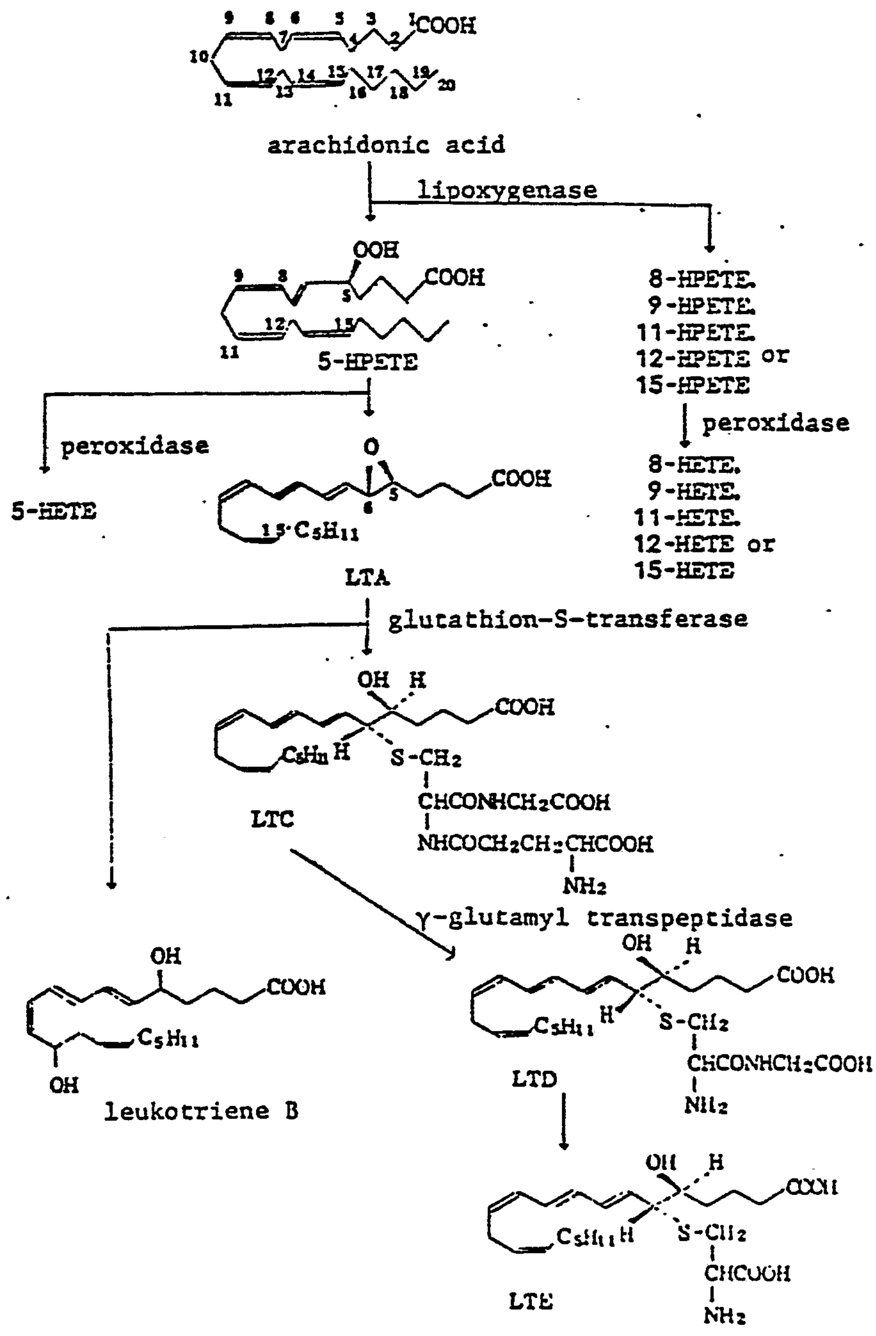


arachidonic acid

5-HPETE

8-HPETE, 9-HPETE, 11-HPETE, 12-HPETE or 15-HPETE

5-HETE

LTA

8-HETE, 9-HETE, 11-HETE, 12-HETE or 15-HETE

LTC

leukotriene B

LTD

LTE

As well known, arachidonic acid is metabolized to various PGs via PG endoperoxides such as $PGG_2$ and $PGH_2$. Arachidonic acid is also metabolized to 5-HPETE, 12-HPETE and 15-HPETE by the action of 5-lipoxygenase, 12-lipoxygenase and 15-lipoxygenase, respectively.

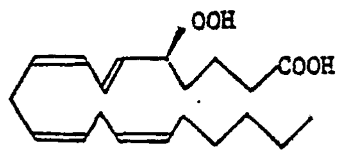

5-HPETE

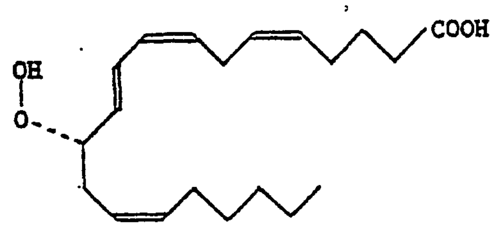

12-HPETE

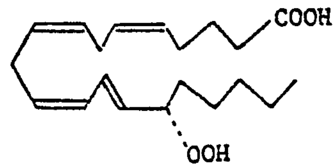

15-HPETE

These HPETE are converted into 5-HETE, 12-HETE and 15-HETE, respectively by the action of peroxidase converting a hydroperoxyl group into a hydroxyl group. Furthermore, LTA is also produced from 5-HPETE. LTA is converted into leukotriene B (abbreviated as LTB hereafter) enzymatically or not enzymatically, or is converted into LTC by the action of glutathion-S-transferase. Further, LTC is converted into LTD by the action of γ-glutamyl transpeptidase.

Moreover, it was recently defined that LTD is metabolized to LTE. [see Biochem. Biophys. Res. Commun., 91, 1266 (1979) and Prostaglandins, 19(5), 645 (1980)]

It was defined that LTC and LTD are the same compound as SRS (slow reacting substance) or SRS-A (slow reacting substance of anaphylaxis) which was well known before. [see Proc. Natl. Acad. Sci. USA, 76, 4275 (1979), Biochem. Biophys. Res. Commun., 91, 1266 (1980), Proc. Natl. Acad. Sci. USA, 77, 2014 (1980) and Nature, 285, 104 (1980)] So it can be understood that the pharmacological characters of these leukotrienes are the same as those of SRS or SRS-A.

Feldberg et al. reported that a substance is released when perfusing cobra venom through isolated lung or incubating cobra venom with vitellus. The substance was named SRS, it has been found that SRS constricts ilem isolated from guinea pig slowly and continually. [see J. Physiol., 94, 187 (1938)]

Moreover, Kellaway et al. showed the relation between SRS-A and allergic reaction from the fact that SRS-A was released when an antigen was sensitized to perfusing lung isolated from guinea pig. [see Quant. J. Exp. Physiol., 30, 121 (1940)]

Brocklehurst reported that when the antigen was sensitized to a lung fragment isolated from a bronchial asthmatics whose specific antigen has been defined, by an operation, histamine and SRS-A were released and they could strongly constrict bronchial muscle. Since such constriction could not be prevented by an antihistaminic agent, he suggested that SRS-A is an important bronchoconstrictor in an asthmatic paroxysm. [see Progr. Allergy, 6, 539 (1962)]

Since then, many reports were published, for instance, SRS-A prepared from human lung slice constrict a tracheal spiral of normal human. [see Int. Arch. Allergy Appl. Immunol., 38, 217 (1970)] When SRS-A prepared from rats or guinea pigs was injected intravenously, significant increase of purlmonory resistance was observed. [see J. Clin. Invest., 53, 1679 (1974)] In addition, a subcataneous injection of SRS-A to guinea pig, rat and monkey could make to enhance vascular permiability. [see Advances in Immunology, 10, 105 (1969), J. Allergy Clin. Immunol., 621, 371 (1978), Prostaglandins, 19(5), 779 (1980) etc.]

Generally, the substance released by immunological reaction is called SRS-A. On the other hand, the substance released by non-immunological reaction such as calcium ionophore is called SRS. However, the above two substances have many similarities each other and, therefore, it is considered they would probably be the same substance.

Based on result of these studies, various leukotrienes biosynthesized from arachidonic acid via LTA (LTC, LTD and LTE, and further other leukotrienes which may be confirmed their structures

hereafter) are considered to be imporatnt factors relating to the appearance of allergic tracheal and bronchial disease, allergic lung disease, allergic shock and various allergic inflammations.

Recently, it was proposed that PG is an important chemical mediator of inflammatory reaction. For example, it was reported that PGE enhance a vascular permiability and a pain. In addition, PGE have a vasodilative action, pyrogenetic action and chemotactic action. [see Makoto Katori et al, Prostaglandin (1978), published by Kohdan-sha] Furhtermore, it was reported that $PGI_2$ alone does not affected vascular permiability, but it enhance vascular permiability of histamine. [see Prostaglandins, 15, 557 (1978)] Moreover, the most recent report indicated that for surpressing PG's action, it is more effective to inhibit not only 5-lipoxygenase but also cyclooxygenase at the same time. [see Biochemcal Pharmacology, 28, 1959 (1979) and European Journal of Pharmacology, 66, 81 (1980)]

As the result of energestic investigations in order to find new compounds inhibiting 5-lipoxygenase,or 5-lipoxygenase and cyclooxygenase at the same time in a living body, and so being effective for prevention and treatment of various diseases induced by not only leukotrienes but also PGs, we have found the compounds which is achieved that purpose, completed this invention.

That is to say, this invention is concerned to 2-aminophenol derivatives of general formula:

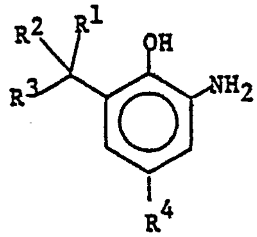

(I)

[wherein $R^1$ represents a straight or branched chain alkyl group containing from 1 to 6 carbon atom(s), a group represented by general formula:

$$-(CH_2)n-COOR^5 \qquad (II)$$

(wherein $R^5$ represents a hydrogen atom or a straight or branched chain alkyl group containing from 1 to 4 carbon atom(s) and n represents zero or an integer from 1 to 6) or a group represented by general formula:

$-(CH_2)m-$ 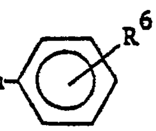

(III)

(wherein $R^6$ represents a hydrogen atom, a halogen atom, a straight or branched chain alkyl group containing from 1 to 4 carbon atom(s), a stright or branched chain alkoxyl group containing from 1 to 4 carbon atom(s), a carbamoyl group, an alkoxycarbonyl group containing from 2 to 6 carbon atoms, a carboxyl group, an amino group, a sulfamoyl group or a cyano group and m represents zero ro an integer of 1 or 2. ), $R^2$ represents a hydroxyl group, a straight or branched chain alkoxyl group containing from 1 to 4 carbon atom(s) or together with $R^3$ an oxo group or a hydroxyimino group. $R^3$ represents a hydrogen atom or together with $R^2$ an oxo group or a hydroxyimino group. $R^4$ represents a halogen atom, a straight or branched chain alkyl group containing from 1 to 6 carbon atom(s) or a phenylthio group. With the exclusion of a compound wherein $R^2$ and $R^3$

taken together represent an oxo group, $R^1$ represents a methyl group and $R^4$ represents a methyl group.]

or their pharmaceutical acceptable acid addition salts.

In the general formula (I), examples of the straight or branched chain alkyl group containing from 1 to 6 carbon atom(s) represented by $R^1$ and $R^4$ are groups of methyl, ethyl, propyl, butyl, pentyl and hexyl and their isomers; preferably, a group of methyl, ethyl, t-butyl or hexyl.

In the general formula (I) and (III), examples of the straight or branched chain alkoxyl group containing from 1 to 4 carbon atom(s) represented by $R^2$ and $R^6$ are groups of methoxyl, ethoxyl, propoxyl, butoxyl and their isomers; preferably, a group of methoxyl or ethoxyl.

In the general formula (II) and (III), examples of the straight or branched chain alkyl group containing from 1 to 4 carbon atom(s) represented by $R^5$ and $R^6$ are groups of methyl, ethyl, propyl, butyl and their isomers; preferably, a group of methyl or ethyl.

In the general formula (III), examples of the group represented by the general formula: $-(CH_2)m-$ are a bond and groups of methylene, ethylene; preferably, a bond.

In the general formula (II) , examples of the group represented by the general formula: $-(CH_2)n-$ are a bond and groups of methylene, ethylene, propylene, butylene, pentylene, hexylene; preferably a propylene group.

In the general formula (III), examples of the alkoxycarbonyl group containing from 2 to 6 carbon atoms represented by $R^6$ are groups of methoxycarbonyl, ethoxycarbonyl, propoxycarbonyl, butoxycarbonyl, pentoxycarbonyl; preferably, a methoxycarbonyl group.

In the general formula (I) and (III), examples of halogen atom represented by $R^4$ and $R^6$ are atoms of chlorine, fluorine, bromine, iodine; preferably, an atom of chlorine or fluorine.

In the general formula (III), it is also preferable the case that $R^6$ represents a hydrogen atom.

In the general formula (I), the following compounds may be listed as preferable compounds:

(i) compounds wherein $R^2$ represents a hydroxyl group, $R^3$ represents a hydrogen atom, $R^1$ represents the group represented by the general formula (III) (wherein $R^6$ represents an atom of chlorine or fluorine or a group of methyl or ethyl and m represents zero), and $R^4$ represents an atom of chlorine or fluorine or a group of t-butyl, methyl or ethyl,

(ii) compounds wherein $R^2$ represents a hydroxyl group, $R^3$ represents a hydrogen atom, $R^1$ represents the group represented by the general formula (III) (wherein $R^6$ represents a group of methoxyl, ethoxyl, methoxycarbonyl or ethoxycarbonyl and m represents zero), and $R^4$ represents an atom of chlorine or fluorine or a group of t-butyl or phenylthio,

(iii) compounds wherein $R^2$ represents a hydroxyl group, $R^3$ represents a hydrogen atom $R^1$ represents a group of ethyl, propyl, butyl, pentyl or hexyl, and $R^4$ represents an atom of chlorine or fluorine or a group of t-butyl, methyl or ethyl,

(iv) compounds wherein $R^2$ represents a group of methoxyl or ethoxyl, $R^3$ represents a hydrogen atom, $R^1$ represents the group represented by the general formula (III) (wherein $R^6$ represents a group of methoxy or

ethoxyl or an atom of chlorine or fluorine and m represents zero), and $R^4$ represents an atom of chlorine or fluorine or a t-butyl group,

(v) compounds wherein $R^2$ and $R^3$ taken together represent a hydroxyimino group, $R^1$ represents the group represented by the general formula (III) (wherein $R^6$ represents an atom of chlorine or fluorine or a group of methoxyl or ethoxyl and m represents zero), and $R^4$ represents an atom of chlorine or fluorine or a t-butyl group,

(vi) compounds wherein $R^2$ and $R^3$ taken together represent an oxo group, $R^1$ represents the group represented by the general formula (III) (wherein $R^6$ represents a hydrogen atom and m represents zero), and $R^4$ represents an atom of chlorine or fluorine or a group of methyl, ethyl or t-butyl,

(vii) compounds wherein $R^2$ and $R^3$ taken together represent an oxo group, $R^1$ represents the group represented by the general formula (III) (wherein $R^6$ represents an atom of chlorine or fluorine or a group of methyl or ethyl, and m represents zero), and $R^4$ represents an atom of chlorine or fluorine or a group of methyl, ethyl or t-butyl,

(viii) compounds wherein $R^2$ and $R^3$ taken together represent an oxo group, $R^1$ represents the group represented by the general formula (III) (wherein $R^6$ represents a group of methoxyl, ethoxyl, methoxycarbonyl or ethoxycarbonyl, and m represent zero), and $R^4$ represents an atom of chlorine or fluorine or a group of phenylthio or t-butyl,

(ix) compounds wherein $R^2$ and $R^3$ taken together represent an oxo group, $R^1$ represents a group of methyl, ethyl, propyl, butyl, pentyl or hexyl, and $R^4$ represents an atom of chlorine or fluorine or a group of t-butyl, methyl or ethyl, and

(x) compounds wherein $R^2$ and $R^3$ taken together represent an oxo group, $R^1$ represents the group represented by the general formula (II) (wherein $R^5$ represents a group of methyl or ethyl, and n represents an integer of 2,3 or 4), and $R^4$ represents a t-butyl group or an atom of chlorine or fluorine.

Recently, an application of compounds similar to this invention was published.[see the British Patent Publication No. 2068960] That is, the specification is concerned to 2-hydroxybenzophenone compounds of general formula:

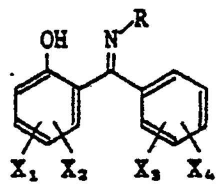

(A)

[wherein each of $X_1$, $X_2$, $X_3$ and $X_4$ represents a hydrogen atom, a halogen atom, an alkyl group of 1 to 4 carbon atom, a trichloromethyl group, a nitro group, a phenyl group, a methoxyl group or an amino group. At least two of them are other than hydrogen atom. And R represents an alkyl group of 1 to 16 carbon atom or an alkenyl group of 2 to 8 carbon atom], their preparation and agents for central nervous system diseases comprising them.

Further, in that specification, it is described that the above compounds may be prepared from 2-hydroxybenzophenone compounds of general formula:

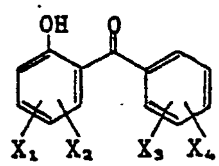

(B)

[wherein all symbols are hereinbefore defined]

However, the above application may not be concidered to be a prior art of our present invention, becauce (i) it is not described in the British application, that the compounds of general formula (A) and (B) have the inhibitory activity on 5-lipoxygenase or cyclooxygenase, which is the significant pharmacological activity for our compounds of the present invention, (ii) the existence of the amino group itself as the substituent $X_1$ or $X_2$ is not essential, but be optional and further

it is not clear where the amino group can be substituted, which in our invention, the amino group is an essential group and is definitely substituted on the o-position of the phenol, and (iii) the symbol R in the formula (A) can not represent a hydroxyl group.

According to a feature of the invention, compounds of the general formula (I) wherein $R^2$ represents a hydroxyl group and $R^3$ represents a hydrogen atom:

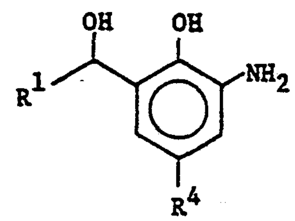

(IA)

(wherein all symbols are as hereinbefore defined)
may be prepared easily by selective reducing the oxo group of the compounds of general formula:

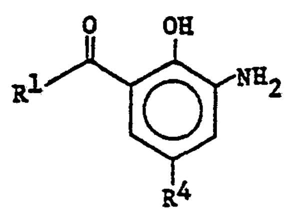

(IB)

(wherein all symbols are as hereinbefore defined) to a hydroxyl group.

The reduction may be carried out, for example, in an atmosphere of hydrogen using a catalyst e.g. palladium-carbon, palladium black in an organic solvent at a temperature from 0°C to 40°C, or using metal hydride e.g. lithium aluminium hydride, sodium borohydride etc., aluminium isopropylalcoholate, an alkaline metal or an alkaline metal amalgam as a catalyst in an alkanol as a hydrogen donor and as a solvent.

Compounds of the general formula (IB) may be prepared by selective reducing of the nitro group of compounds of the general formula:

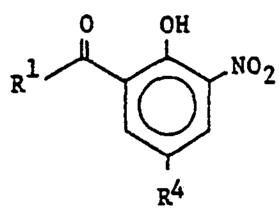

(IV)

(wherein all symbols are as hereinbefore defined) to an amino group.

The reduction may be carried out, for example, by using hydrogen gas in the presence of a catalyst such as nickel, platinum or palladium-carbon, by using iron, zinc, tin or stannous chloride in addition to hydrochloric acid, by using titanium trichloride in the presence of benzene or a mixture of benzene and tetrahydrofuran, or by using sodium sulfide or ammonium sulfide in water or an aqueous alcohol, or sodium hydrosulfite in ammonia water etc.

Compounds of the general formula (IV) may be prepared easily by nitration of compounds of general formula:

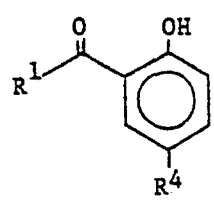

(V)

(wherein all symbols are hereinbefore defined)

The nitration may be carried out using a conventional nitrating reagent, for example, a mixture of nitric acid and sulfuric acid, a mixture of nitric acid and sulfuric acid in acetic acid, or nitric acid in acetic acid.

Compounds of the general formula (V) may be prepared by subjecting to;

(A) Fries rearrengement reaction of ester compounds of general formula:

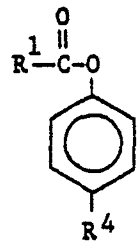

(VI)

(wherein all symbols are as hereinbefore defined) or

(B) Acylation of Friedel-Crafts reaction of compounds of general formula:

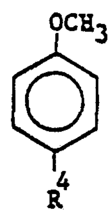

(VII)

(wherein all symbols are as hereinbefore defined)

and compounds of general formula:

$$R^1—CO—X \quad \text{(VIII)}$$

(wherein $R^1$ represents as hereinbefore defined and X represents a halogen atom)

to obtain compounds of general formula:

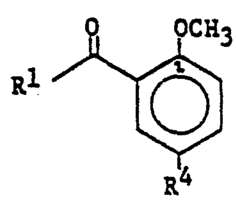

(IX)

(wherein all symbols are hereinbefore defined)

and converting a methoxyl group at the 1-position of compounds of the general formula (IX) to a hydroxyl group.

Fries rearrengement reaction as (A) have been known well, for example, it may be carried out at the presence of a catalyst of Friedel-Crafts reaction e.g. alminium chloride, titanium chloride, in an inert solvent e.g. methylene chloride or without solvent; preferably, without solvent heating at a temperature from 80°C to 200°C.

Acylation of Friedel-Crafts reaction as (B) also have been known well, at the presence of a catalyst e.g. aluminium chloride, aluminium bromide, stannic chloride, ferric chloride, boron trifluoride, phosphorus pentachloride etc. in an inert organic solvent e.g. carbon disulfide, nitrobenzene, chloroform, methylene chloride, carbon tetrachloride, tetrahydrofuran etc. at a temperature from -20°C to 50 °C.

Generally, products of Friedel-Crafts reaction are a mixture of compounds of the general formula (IX) and compounds of the general formula (V), but compounds of the general formula (V) only may be selectively obtained according to the kind of the groups represented by $R^1$ and $R^4$ or according to the reaction condition.

Converting method from a methoxyl group to a hydroxyl group have been known well, for example, using hydrobromic acid or a mixture of hydrobromic acid and hydroiodic acid in acetic acid or without solvent, with refluxing.

Compounds of the general formula (IV) wherein $R^1$ represents the group represented by the general formula (III) and $R^6$ represents an alkoxycarbonyl group containing from 2 to 6 carbon atoms may be prepared by esterification of compounds of the general formula (IV) wherein $R^1$ represents the group represented by the general formula (III) and $R^6$

represents a carboxyl group.

Also, compounds of the general formula (IV) wherein $R^1$ represents the group represented by the general formula (II) and $R^5$ represents a straight or branched chain alkyl group may be prepared by esterification using compounds of the general formula (IV) wherein $R^1$ represents the group represented by the group general formula (II) and $R^5$ represents a hydrogen atom.

Esterification reaction have been known, it may be carried out using a diazoalkane or an alkyl halide.

In the case by Fries rearrengement, the compounds of the general formula (VI) may be prepared by reacting compounds of general formula:

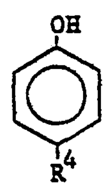

(X)

(wherein all symbols are as hereinbefore defined)
and the compounds of the general formula (VIII).

The compounds of the general formula (VII), (VIII) and (X) are compounds known *per se* or may be prepared by method known *per se*.

Moreover, according to a feature of the present invention, the compouds of general formula (I) wherein $R^2$ and $R^3$ taken together represent a hydroxyimino group i.e. general formula:

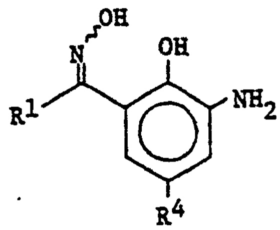

(IC)

(wherein all symbols are as hereinbefore defined)
may be prepared by subjecting to oxime forming reaction of the compounds of the general formula (IB).

Method of oxime forming reaction have been known well, it may be carried out using a hydroxyamine hydrochloride, at the presence of sodium carbonate or potassium carbonate, in an inert organic solvent e.g. methanol or ethanol, at a temperature from room to refluxing of the solvent.

Further, according to a feature of the present invention, the compouds of general formula (I) wherein $R^2$ represents a straight or branched chain alkoxyl group and $R^3$ represents a hydrogen atom i.e. general formula:

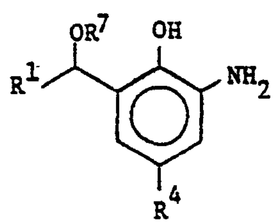

(ID)

(wherein $R^7$ represents a straight or branched chain alkyl group containing from 1 to 4 carbon atom(s), and the other symbols are as hereinbefore defined)
may be prepared by converting reaction the hydroxyl group of compounds

of the general formula (IA) to an alkoxyl group.

The converting method is well known, for example, by using an acid such as hydrochloric acid, nitric acid, p-toluenesulfonic acid etc. as a catalyst, in corresponding alkanol such as methanol, ethanol, propanol, butanol etc. at a temperature from 0°C to refluxing of the alkanol.

Acid addition salts of 2-aminophenol derivatives of the general formula (I) may be prepared from the compounds of the general formula (I) by methods known per se, for example, by reacting stoichiometric quantities of a compound of the general formula (I) and an appropriate acid e.g. an inorganic acid such as hydrochloric acid, hydrobromic acid, hydroiodic acid, sulfuric acid, phosphoric acid or nitric acid, or an organic acid such as acetic acid, lactic acid, tartric acid, benzoic acid, citric acid, methanesulphonic acid, ethanesulphonic acid, benzenesulphonic acid, toluenesulphonic acid or isethionic acid, in a suitable solvent.

Preferably, acid addition salts are non-toxic salts. By the term 'non-toxic' as used in this specification, is meant salts the anions of which are relatively innocuous to animal organism when used in thrapeutic doses so that the benefitical pharmacological properties of the compounds of genral formula (I) are not vitiated by side effects ascrible to those anions. Preferably the salts are water-soluble. Suitable acid addition salts of 2-aminophenol derivatives are, for

example, inorganic acid salts such as hydrochloride, hydrobromide, hydroiodide, sulfate,phosphorate, nitrate, or orgenic acid salts such as acetate, lactate, tartrate,benzoate, citrate, methanesulphonate, ethanesulphonate, benzenesulphonate, toluenesulphonate or isethionate.

Compounds represented by the general formula (I) and their pharmaceutical acceptable acid addition salts possess an inhibitory effect on 5-lipoxygenase specifically, or 5-lipoxygenase and cyclooxygenase at the same time, and therefore, effective when it is desired to control the biosynthesis of leukotrienes and various PGs in mammals include humans, especially humans.

For example, in standard assay system using polymorphonuclear leukocytes from guinea pig (details of experiments were described hereafter), the concentrations for 50% inhibitions on 5-lipoxygenase and on cyclooxygenase by (a) 3-amino-5-t-butyl-4'-chloro-2-hydroxybenzhydrol hydrochloride were 1 μM and 100 μM, respectively, (b) 3-amino-4'-chloro-5-fluoro-2-hydroxybenzhydrol hydrochloride were 1 μM and 7.2 μM, respectively, (c) 3-amino-5,4'-dichloro-2-hydroxybenzophenone were 1 μM and 3 μM, respectively, (d) 3-amino-4'-chloro-5-fluoro-2-hydroxybenzophenone were 1 μM and 2 μM, respectively, therefore, it is comfirmed that compound (a) specifically inhibits 5-lipoxygenase at a low concentration, and compounds (b), (c) and (d) inhibit both enzymes at the same time at a very low concentration.

The experiments for screening using polymorphonuclear leukocytes was carried out as follows: The reaction mixture (0.1 ml) containing

5 µmole of potassium phosphate at pH 7.4, 0.1 µmole of calsium chloride, polymorphonuclear leukocytes prepared from guinea pigs (4 x $10^7$ cells) and test compound was preincubated for 5 minutes at 30°C. The reaction was started by the addition of [$^{1-14}C$] arachidonic acid (2.2 x $10^5$ dpm, 1.8 nmole), followed by the incubation at 30°C for 5 minutes. The reaction was terminated by the addition of 0.3 ml of mixture of ether/methanol/1M citric acid (30/4/1). The organic phase (20 µl) was applied to silica gel plate. Thin layer chromatography was performed to separate each product with solvent system of ether/petroleum ether/acetic acid (85/15/0.1).

Activity of 5-lipoxygenase and cyclooxygenase were calculated from the measurement of the radioactivity corresponding to 5-HETE and 12L-hydroxy-5,8,10-heptareienoic acid (i.e. HHT) or thromboxane $B_2$ (i.e. $TXB_2$). [it have been known that HHT and $TXB_2$ were the last products of the system concerning cyclooxygenase in the arachidonate cascade] Further, it was determined that the concentrations of 50% inhibitions to produce 5-HETE and HHT or $TXB_2$ for the 50% inhibiting concentrations of 5-lipoxygenase and cyclooxygenase, respectively.

Further, in an other assay system measuring the inhibitory activity of test compound on 5-lipoxygenase only was evaluated by using an enzyme prepared from polymorphonuclear leukocytes of guinea pig. Guinia pig was treated by casein and harvested polymorphonuclear leukocytes from cavity were sonicated, followed by the centrifugation at 105,000 x g. The supernate was used as an enzyme throughout this work. The supernate (100 - 500 µg of protein) was incubated for 5 minutes at 30°C with [$^{1-14}C$] arachidonic acid and 1 mM

calsium chloride in the presence of absence of test compound. The reaction was terminated by the addition of ether. Ether extract was separated by thin layer chromatography using silica gel plates to observe production of 5-HETE, 5,12-diHETE and LTB.

In this assay, the concentrations of 2-amino-4-t-butyl-6-propionylphenol hydrochloride, 3-amino-5-t-butyl-2-hydroxybenzophenone hydrochloride, 3-amino-5-t-butyl-4'-chloro-2-hydroxybenzophenone hydrochloride and 2-amino-4-t-butyl-6-heptanoylphenol hydrochloride required to produce a 50% inhibition on the productions of 5-HETE, 5,12-diHETE and leukotriene B (concentration for a 50% inhibition of 5-lipoxygenase, i.e.$IC_{50}$ ) were 5 μM, 2.6 μM, 0.17 μM and 2 μM, respectively.

On the other hand, all extent of the compounds of the present invention were confirmed that their acute toxicities were more than 30 mg/Kg by intravenous injection. Therefore, the compounds of the present invention may be considered to be sufficiently safe and suitable for medical use.

For example, in a test of acute toxicity using mice and administering by intravenous injection to their tail vein, 3-amino-5-chloro-2-hydroxy-4'-methoxybenzophenone hydrochloride and 2-amino-4-t-butyl-6-(1-hydroxyheptyl)phenol possessed no dead case in 30 cases at doses of 10 mg/Kg and 30 mg/Kg.

To control the biosynthesis of leukotrienes and prostaglandins are useful for the prevention and the treatment of allergic tracheal and bronchial diseases or allergic lung diseases, allergic shocks or various allergic inflammations in addition that various inflammations induced by prostaglandins in mammals include humans, especially humans. For such purpose, the compounds of this invention are usually administered systemically, for example, orally, rectally or parenterally. Doses are determined depending upon age, symptoms, the desired therapeutic effects, the route of administration, the duration of the treatment and the like, and are generally and preferably about 0.1 mg to 500 mg for oral administration, and 1 µg to 1 mg for intravenous injection of 0.1 µg to 0.1 mg/hour for intravenous infusion, specially when required emargency treatment.

Solid compositions for oral administration include compressed tablets, pills, dispersible powders and granules. In such solid compositions, one or more of the active compounds is or are, admixed with at least one inert diluent such as calcium carbonate, potato starch, dextrin, alginic acid, lactose, mannitol, glucose and cacao butter. The compositions may also comprise, as is normal practice, additional substances other than inert diluents e.g. lubricating agents such as magnesium stearate. The tablets or pill may, if desired, be coated and made into sugar-coated, gelatin-coated, euteric-coated or film-coated tablets and pills, or tablets or pills may be coated with two or more layers. Liquid compositions for oral administration include pharmaceutically acceptable emulsions, solutions, suspentions,

syrups and elixirs containing inert diluents commonly used in the art, such as water or liquid paraffin. Besides inert diluents such compositions may also comprise adjuvants such as wetting and suspending agents, and sweetening, flavouring, perfuming and preservating agents.

The compositions according to this invention for oral administration, also include capsules of absorbable materials such as gelatin containing one or more of the active substances with or without the addition of diluents or excipients.

Solid compositions for intrarectal administration include suppositories formulated in manner known per se and containing one or more of the active compounds.

Preparation according to this invention for parenteral administration include sterile aqueous or non-aqueous solutions, suspensions or emulsions. Example of non-aqueous solvents or suspending media are propylene glycol, polyethylene glycol, ethanol, vegetable oils such as olive oil, and injectable organic esters such as ethyl oleate or sorbitan esters. These compositions may also include adjuvants such as preserving, wetting, emulsifying and dispersing agents. They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

The percentage of active ingredient in the compositions of the present invention may be varied, it being necessary that it should constitute a proportion such that a suitable dosage for the desired

therapeutic effect shall be obtained. Several unit dosage forms may of course be administered at the same time. In general the preparations should normally contain at least 0.025% by weight of active substance when required for administeration by injection: for oral administration the preparations will normally contain at least 0.1% by weight of active substance.

Preferable 2-aminophenol derivatives included this invention, for example, the following compounds and their acid addition salts may be listed:

3-amino-5,4'-dichloro-2-hydroxybenzhydrol,
3-amino-4'-chloro-5-fluoro-2-hydroxybenzhydrol,
3-amino-5-t-butyl-4'-chloro-2-hydroxybenzhydrol,
3-amino-4'-chloro-2-hydroxy-5-methylbenzhydrol,
3-amino-4'-chloro-5-ethyl-2-hydroxybenzhydrol,
3-amino-5-chloro-4'-fluoro-2-hydroxybenzhydrol,
3-amino-5,4'-difluoro-2-hydroxybenzhydrol,
3-amino-5-t-butyl-4'-fluoro-2-hydroxybenzhydrol,
3-amino-4'-fluoro-2-hydroxy-5-methylbenzhydrol,
3-amino-5-ethyl-4'-fluoro-2-hydroxybenzhydrol,
3-amino-5-chloro-2-hydroxy-4'-methylbenzhydrol,
3-amino-5-fluoro-2-hydroxy-4'-methylbenzhydrol,
3-amino-5-t-butyl-2-hydroxy-4'-methylbenzhydrol,
3-amino-2-hydroxy-5,4'-dimethylbenzhydrol,
3-amino-5-ethyl-2-hydroxy-4'-methylbenzhydrol,
3-amino-5-chloro-4'-ethyl-2-hydroxybenzhydrol,
3-amino-4'-ethyl-5-fluoro-2-hydroxybenzhydrol,
3-amino-5-t-butyl-4'-ethyl-2-hydroxybenzhydrol,

3-amino-4'-ethyl-2-hydroxy-5-methylbenzhydrol,

3-amino-5,4'-diethyl-2-hydroxybenzhydrol,

3-amino-5-chloro-2-hydroxy-4'-methoxybenzhydrol,

3-amino-5-fluoro-2-hydroxy-4'-methoxybenzhydrol,

3-amino-2-hydroxy-4'-methoxy-5-phenylthiobenzhydrol,

3-amino-5-t-butyl-2-hydroxy-4'-methoxybenzhydrol,

3-amino-5-chloro-4'-ethoxy-2-hydroxybenzhydrol,

3-amino-4'-ethoxy-5-fluoro-2-hydorxybenzhydrol,

3-amino-4'-ethoxy-2-hydroxy-5-phenylthiobenzhydrol,

3-amino-5-t-butyl-4'-ethoxy-2-hydroxybenzhydrol,

3-amino-5-chloro-2-hydroxy-4'-methoxybenzhydrol,

3-amino-5-fluoro-2-hydroxy-4'-methoxycarbonylbenzhydrol,

3-amino-2-hydroxy-4'-methoxycarbonyl-5-phenylthiobenzhydrol,

3-amino-5-t-butyl-2-hydroxy-4'-methoxycarbonylbenzhydrol,

3-amino-5-chloro-4'-ethoxycarbonyl-2-hydroxybenzhydrol,

3-amino-4'-ethoxycarbonyl-5-fluoro-2-hydroxybenzhydrol,

3-amino-4'-ethoxycarbonyl-2-hydroxy-5-phenylthiobenzhydrol,

3-amino-5-t-butyl-4'-ethoxycarbonyl-2-hydroxybenzhydrol,

2-amino-4-chloro-6-(1-hydroxypropyl)phenol,

2-amino-4-fluoro-6-(1-hydroxypropyl)phenol,

2-amino-4-t-butyl-6-(1-hydroxypropyl)phenol,

2-amino-6-(1-hydroxypropyl)-4-methylphenol,

2-amino-4-ethyl-6-(1-hydroxypropyl)phenol,

2-amino-4-chloro-6-(1-hydroxybutyl)phenol,

2-amino-4-fluoro-6-(1-hydorxybutyl)phenol,

2-amino-4-t-butyl-6-(1-hydroxybutyl)phenol,

2-amino-6-(1-hydroxybutyl)-4-methylphenol,

2-amino-4-ethyl-6-(1-hydroxybutyl)phenol,

2-amino-4-chloro-6-(1-hydroxypentyl)phenol,

2-amino-4-fluoro-6-(1-hydroxypentyl)phenol,

2-amino-4-t-butyl-6-(1-hydroxypentyl)phenol,

2-amino-6-(1-hydroxypentyl)-4-methylphenol,

2-amino-4-ethyl-6-(1-hydroxypentyl)phenol,

2-amino-4-chloro-6-(1-hydroxyhexyl)phenol,

2-amino-4-fluoro-6-(1-hydroxyhexyl)phenol,

2-amino-4-t-butyl-6-(1-hydroxyhexyl)phenol,

2-amino-6-(1-hydroxyhexyl)-4-methylphenol,

2-amino-4-ethyl-6-(1-hydroxyhexyl)phenol,

2-amino-4-chloro-6-(1-hydroxyheptyl)phenol,

2-amino-4-fluoro-6-(1-hydroxyheptyl)phenol,

2-amino-4-t-butyl-6-(1-hydroxyheptyl)phenol,

2-amino-6-(1-hydroxyheptyl)-4-methylphenol,

2-amino-4-ethyl-6-(1-hydroxyheptyl)phenol,

2-amino-4-chloro-6-(α-ethoxy-4-methoxybenzyl)phenol,

2-amino-6-(α-ethoxy-4-methoxybenzyl)-4-fluorophenol,

2-amino-4-t-butyl-6-(α-ethoxy-4-methoxybenzyl)phenol,

2-amino-4-chloro-6-(α,4-diethoxybenzyl)phenol,

2-amino-6-(α,4-diethoxylbenzyl)-4-fluorophenol,

2-amino-4-t-butyl-6-(α,4-diethoxybenzyl)phenol,

2-amino-4-chloro-6-(α-ethoxy-4-chlorobenzyl)phenol,

2-amino-6-(α-ethoxy-4-chlorobenzyl)-4-fluorophenol,

2-amino-4-t-butyl-6-(α-ethoxy-4-chlorobenzyl)phenol,

2-amino-4-chloro-6-(α-ethoxy-4-fluorobenzyl)phenol,

2-amino-6-(α-ethoxy-4-fluorobenzyl)-4-fluorophenol,

2-amino-4-t-butyl-6-(α-ethoxy-4-fluorobenzyl)phenol,

2-amino-4-chloro-6-(α,4-dimethoxybenzyl)phenol,

2-amino-6-(α,4-dimethoxybenzyl)-4-fluorophenol,

2-amino-4-t-butyl-6-(α,4-dimethoxybenzyl)phenol,

2-amino-4-chloro-6-(α-methoxy-4-ethoxybenzyl)phenol,

2-amino-6-(α-methoxy-4-ethoxybenzyl)-4-fluorophenol,

2-amino-4-t-butyl-6-(α-methoxy-4-ethoxybenzyl)phenol,

2-amino-4-chloro-6-(α-methoxy-4-chlorobenzyl)phenol,

2-amino-6-(α-methoxy-4-chlorobenzyl)-4-fluorophenol,

2-amino-4-t-butyl-6-(α-methoxy-4-chlorobenzyl)phenol,

2-amino-4-chloro-6-(α-methoxy-4-fluorobenzyl)phenol,

2-amino-6-(α-methoxy-4-fluorobenzyl)-4-fluorophenol,

2-amino-4-t-butyl-6-(α-methoxy-4-fluorobenzyl)phenol,

3-amino-5,4'-dichloro-2-hydroxybenzophenoneoxime,

3-amino-4'-chloro-5-fluoro-2-hydroxybenzophenoneoxime,

3-amino-5-t-butyl-4'-chloro-2-hydroxybenzopheneneoxime,

3-amino-5-chloro-4'-fluoro-2-hydorxybenzophenoneoxime,

3-amino-5,4'-difluoro-2-hydroxybenzophenoneoxime,

3-amino-5-t-butyl-4'-fluoro-2-hydroxybenzophenoneoxime,

3-amino-5-chloro-2-hydroxy-4'-methoxybenzophenoneoxime,

3-amino-5-fluoro-2-hydorxy-4'-methoxybenzophenoneoxime,

3-amino-5-t-butyl-2-hydorxy-4'-methoxybenzophenoneoxime,

3-amino-5-chloro-4'-ethoxy-2-hydroxybenzophenoneoxime,

3-amino-4'-ethoxy-5-fluoro-2-hydroxybenzophenoneoxime,

3-amino-5-t-butyl-4'-ethoxy-2-hydorxybenzophenoneoxime,

3-amino-5-chloro-2-hydroxybenzophenone,

3-amino-5-fluoro-2-hydroxybenzophenone,

3-amino-5-t-butyl-2-hydroxybenzophenone,

3-amino-2-hydroxy-5-methylbenzophenone,

3-amino-5-ethyl-2-hydroxybenzophenone,

3-amino-5,4'-dichloro-2-hydroxybenzophenone,

3-amino-4'-chloro-5-fluoro-2-hydorxybenzophenone,

3-amino-5-t-butyl-4'-chloro-2-hydroxybenzophenone,

3-amino-4'-chloro-2-hydroxy-5-methylbenzophenone,

3-amino-4'-chloro-5-ethyl-2-hydroxybenzophenone,

3-amino-5-chloro-4'-fluoro-2-hydroxybenzophenone,

3-amino-5,4'-difluoro-2-hydroxybenzophenone,

3-amino-5-t-butyl-4'-fluoro-2-hydroxybenzophenone,

3-amino-4'-fluoro-2-hydroxy-5-methylbenzophnone,

3-amino-5-ethyl-4'-fluoro-2-hydorxybenzophenone,

3-amino-5-chloro-2-hydroxy-4'-methylbenzophenone,

3-amino-5-fluoro-2-hydroxy-4'-methylbenzophenone,

3-amino-5-t-butyl-2-hydorxy-4'-methylbenzophenone,

3-amino-2-hydroxy-5,4'-dimethylbenzophenone,

3-animo-5-ethyl-2-hydroxy-4'-methylbenzophenone,

3-amino-5-chloro-4'-ethyl-2-hydroxybenzophenone,

3-amino-4'-ethyl-5-fluoro-2-hydroxybenzophenone,

3-amino-5-t-butyl-4'-ethyl-2-hydroxybenzophenone,

3-amino-4'-ethyl-2-hydroxy-5-methylbenzophenone,

3-amino-5,4'-diethyl-2-hydroxybenzophenone,

3-amino-5-chloro-2-hydroxy-4'-methoxybenzophenone,

3-amino-5-fluoro-2-hydroxy-4'-methoxybenzophenone,

3-amino-2-hydroxy-4'-methoxy-5-phenylthiobenzophenone,

3-amino-5-t-butyl-2-hydroxy-4'-methoxybenzophenone,

3-amino-5-chloro-4'-ethoxy-2-hydorxybenzophenone,

3-amino-4'-ethoxy-5-fluoro-2-hydroxybenzophenone,

3-amino- 4'-ethoxy-2-hydroxy-5-phenylthiobenzophenone,

3-amino-5-t-butyl-4'-ethoxy-2-hydroxybenzophenone,

3-amino-5-chloro-2-hydroxy-4'-methoxycarbonylbenzophenone,

3-amino-5-fluoro-2-hydroxy-4'-methoxycarbonylbenzophenone,

3-amino-2-hydroxy-4'-methoxycarbonyl-5-phenylthiobenzophenone,

3-amino-5-t-butyl-2-hydroxy-4'-methoxycarbonylbenzophenone,

3-amino-5-chloro-4'-ethoxycarbonyl-2-hydroxybenzophenone,

3-amino-4'-ethoxycarbonyl-5-fluoro-2-hydroxybenzophenone,

3-amino-4'-ethoxycarbonyl-2-hydroxy-5-phenylthiobenzophenone,

3-amino-5-t-butyl-4'-ethoxycarbonyl-2-hydrocybenzophenone,

2-acetyl-6-amino-4-chlorophenol,

2-acetyl-6-amino-4-fluorophenol,

2-acetyl-6-amino-4-t-butylphenol,

2-amino-4-chloro-6-propionylphenol,

2-amino-4-fluoro-6-propionylphenol,

2-amino-4-t-butyl-6-propionylphenol,

2-amino-6-butyryl-4-chlorophenol,

2-amino-6-butyryl-4-fluorophenol,

2-amino-4-t-butyl-6-butyrylphenol,

2-amino-4-chloro-6-pentanoylphenol,

2-amino-4-fluoro-6-pentanoylphenol,

2-amino-4-t-butyl-6-pentanoylphenol,

2-amino-4-chloro-6-hexanoylphenol,

2-amino-4-fluoro-6-hexanoylphenol,

2-amino-4-t-butyl-6-hexanoylphenol,

2-amino-4-chloro-6-heptanoylphenol,

2-amino-4-fluoro-6-heptanoylphenol,

2-amino-4-t-butyl-6-heptanoylphenol,

2-amino-4-t-butyl-6-(2-methoxycarbonylacetyl)phenol,

2-amino-4-chloro-6-(2-methoxycarbonylacetyl)phenol,

2-amino-4-fluoro-6-(2-methoxycarbonylacetyl)phenol,

2-amino-4-t-butyl-6-(3-methoxycarbonylpropionyl)phenol,

2-amino-4-chloro-6-(3-methoxycarbonylpropionyl)phenol,

2-amino-4-fluoro-6-(3-methoxycarbonylpropionyl)phenol,

2-amino-4-t-butyl-6-(4-methoxycarbonylbutyryl)phenol,

2-amino-4-chloro-6-(4-methoxycarbonylbutyryl)phenol,

2-amino-4-fluoro-6-(4-methoxycarbonylbutylryl)phenol,

2-amino-4-t-butyl-6-(2-ethoxycarbonylacetyl)phenol,

2-amino-4-chloro-6-(2-ethoxycarbonylacetyl)phenol,

2-amino-6-(2-ethoxycarbonylacetyl)-4-fluorophenol,

2-amino-4-t-butyl-6-(3-ethoxycarbonylpropionyl)phenol,

2-amino-4-chloro-6-(3-ethoxycarbonylpropionyl)phenol,

2-amino-6-(3-ethoxycarbonylpropionyl)-4-fluorophenol,

2-amino-4-t-butyl-6-(4-ethoxycarbonylbutyryl)phenol,

2-amino-4-chloro-6-(4-ethoxycarbonylbutyryl)phenol,

2-amino-6-(4-ethoxycarbonylbutyryl)-4-fluorophenol.

The following Reference Examples and Examples illustrate, but not limit, the preparation of compounds of the present invention. In tye Reference Examples and Examples, 'TLC'. 'IR', 'NMR' and 'Mass' represent 'Thin layer chromatography', 'Infrared absorption spectrum', 'Nuclear magnetic resonance' and 'Mass spectrum', respectively. Where

solvent ratios are specified in chromatographic separations, the ratios are by volume: the solvents in parentheses in thin layer chromatography show the developing solvent used. Except when specified otherwise, infrared spectra are recorded by the KBr tablet method and nuclear magnetic resonance spectra are recorded in deuterochloroform ($CDCl_3$) solution.

Reference Example 1.

4-Chloro-benzoic acid 4-chlorophenyl ester.

To the solution of 2.8 g of 4-chlorophenol in 50 ml of methylene chloride was added 2.2 ml of triethylamine, cooled with ice bath, and added dropwise 3.8 g of 4-chlorobenzoyl chloride at 5 °C. After addition, ice bath was removed, and the reaction mixture was stirred at room temperature for 30 minutes. To the reaction mixture was added water to dissolve salts, the oil layer was washed with successive, 2N hydrochloric acid, a saturated aqueous solution of sodium hydrocarbonate and a saturated aqueous solution of sodium chloride, dried over anhydrous sodium sulfate, and concentrated under reduced pressure to give 5.87 g of the title compound as crude crystals having the following physical characteristics:

NMR: $\delta$=8.00(2H,d), 7.36(2H,d), 7.26(2H,d), 7.10(2H,d);

IR: $\nu$=3095, 1740, 1590, 1480, 1400, 1285, 1270, 1205
1170, 1160, 1090, 1075, 1010, 870, 845, 815
800, 750 $cm^{-1}$;

Mass: m/e = 266($M^+$), 139, 110.

By the procedure as described in Reference Example 1, the following compounds were given:

(a) 4-Chloro-benzoic acid 4-fluorophenyl ester.

(Instead of 4-chlorophenol using in Reference Example 1, 2.46 g of 4-fluorophenol was used.)

Yield: 5.46 g;

NMR: $\delta$=7.98(2H,d), 7.38(2H,d), 7.3 - 6.9(4H,m);

IR: $\nu$=3100, 1730, 1590, 1500, 1400, 1295, 1280, 1240 1190, 1090, 1080, 1070, 1010, 875, 855 $cm^{-1}$;

Mass: m/e = 250($M^+$), 173, 139, 111.

(b) Heptanoic acid 4-chlorophenyl ester.

(Instead of 4-chlorobenzoylchloride using in Reference Example 1, 3.27 g of heptanoyl chloride was used.)

Yield: 5.9 g;

NMR: $\delta$=7.9(2H,d), 7.2(2H,d), 2.4(2H,t);

IR: $\nu$=3090, 1735, 1590 $cm^{-1}$;

Mass: m/e = 242, 240($M^+$).

(c) 4-Methoxybenzoic acid 4-phenylthiophenyl ester.

Starting material: 1 g of 4-phenylthiophenol and 853 mg of 4-methoxybenzoic acid;

Yield: 760 mg;

TLC(Methlene chloride:hexane = 2:1): Rf = 0.6;

NMR: $\delta$ = 8.0(2H,d), 7.5 - 6.7(11H,m), 3.80(3H,s);

Mass: m/e = 336($M^+$), 201, 135, 107, 92.

Reference Example 2.

5,4'-Dichloro-2-hydroxybenzophenone.

A mixture of the ester compound (prepared in Reference Example 1.) and 2.8 g of titanium tetrachloride was stirred at 150 °C for 18 hours, to the reaction mixture was added 50 ml of methylene chloride to quench solids with cooling with ice bath, moreover, added water and 6N hydrochloric acid, stirred well, and then separated.

The oil layer was washed with a saturated aqueous solution of sodium chloride, dried over anhydrous sodium sulfate and concentrated under reduced pressure. The residue was purified by column chromatography on silica gel using a mixture of methylene chloride and n-hexane (2:3) as an eluent to give 1.4 g of the title compound as yellow crystals having the following physical characteristics:

TLC(Methylene chloride:n-hexane=1:1) : Rf=0.6;

NMR: δ=11.63(1H,s), 7.8∿7.2(5H,m), 6.93(2H,d);

IR : ν=3400, 3030, 1630, 1595, 1460, 1400, 1325, 1285 1235, 1220, 1175, 1090, 1010, 955, 945, 900, 845, 830, 785 $cm^{-1}$;

Mass: m/e = 266($M^+$), 230, 154, 139, 111, 99.

By the procedure as described in Reference Example 2, the following compounds were given:

(a) 4'-Chloro-5-fluoro-2-hydroxybenzophenone.

(Instead of the ester compound using in Reference Example 2, 4.47 g of the ester compound prepared in Reference Example 1(a) was used.)

Yield: 3.64 g;

TLC(Methylene chloride:n-hexane=1:1): Rf=0.44;

NMR: δ=11.45(1H,s), 7.6∿6.6(7H,m);

IR : ν=3440, 3080, 1635, 1600, 1585, 1560, 1470, 1420, 1395 1340, 1285, 1250, 1230, 1180, 1125, 1095, 1015, 975, 890, 870, 840, 825, 790 $cm^{-1}$;

Mass: m/e = 250($M^+$), 215, 139, 111.

(b) 4-Chloro-2-heptanoylphenol.

(Instead of the ester compound using in Reference Example 2, 1.8 g of the ester compound prepared in Reference Example 1 (b) was used and reaction was carried out at 100 °C.)

Yield: 1.3 g;

NMR: δ=11.8(1H,s), 7.7(1H,d), 7.5(1H,dd), 6.8(1H,d), 2.9(2H,t);

Mass: m/e = 242, 240($M^+$).

(c) 2-Hydroxy-4'-methoxy-5-phenylthiobenzophenone.

Starting material: 7.4 g of 4-methoxybenzoic acid 4-phenylthiophenyl ester (prepared in Reference Example 1(c));

Yield: 1.2 g;

TLC(Methylene chloride:hexane = 1:1): Rf = 0.3;

NMR: δ = 12.06(1H,s), 7.74(1H,d), 7.63(2H,d), 7.57(1H,dd), 7.22(5H,s), 7.05(1H,d), 6.93(2H,d), 3.88(3H,s);

IR(Liquid film): ν = 3050, 3010, 2970, 2930, 2840, 1620, 1600, 1510, 1465, 1330, 1260, 1170, 1030, 960, 845, 795, 690 $cm^{-1}$;

Mass: m/e = 336($M^+$), 228, 171, 135, 92.

Reference Example 3.

5-Chloro-2-hydroxy-4'-methoxybenzophenone.

In an atomosphere of nitrogen, 1.28 g of aluminium chloride and 50 ml of methylene chloride was mixed and stirred for 15 minutes. The mixture was cooled to 0 °C, added dropwise 13.64 g of p-methoxybenzoyl chloride, and stirred for 15 minutes at room

temperature. To the reaction solution was added dropwise 10 ml of p-chloroanisol dissolved in 50 ml of methylene chloride and stirred for 18 hours. The reaction mixture was poured into a mixture of ice-water and hydrochloric acid and extracted with methylene chloride. The extract was washed with an aqueous solution of sodium hydrocarbonate, dried over anhydrous sodium sulfate, and concentrated under reduced pressure. The residue was recrystallized from cyclohexane to give 8.6 g of the title compound having the following physical characteristics:

TLC (Benzene): Rf=0.46;

IR : ν=1620, 1590, 1560, 1460, 1325, 1305, 1250, 1230, 1210, 1165, 1020, 955, 940, 840, 830, 785 $cm^{-1}$;

NMR: δ=11.82(1H, s), 7.9-6.9(7H, m), 3.92(3H, s);

Mass: m/e = 262($M^+$).

By the procedure as described in Reference Example 3, the following compounds were given:

(a) 5-Chloro-2-methoxy-4'-methoxycarbonylbenzophenone.

Starting material: 25 g of p-Methoxycarbonylbenzoyl chloride;

Yield: 3.1 g;

Melting point: 99 - 100 °C;

TLC (Benzene): Rf=0.18;

IR : ν=1715, 1650, 1475, 1400, 1260, 1100, 815 $cm^{-1}$;

NMR: δ=8.12(2H, d), 7.83(2H, d), 7.3-7.6(2H, m), 6.95(1H, d), 3.94(3H, s), 3.67(3H, s);

Mass: m/e = 304($M^+$), 287, 273, 245, 169.

(b) 1-t-Butyl-3-(4-carboxybutanoyl)-4-methoxybenzene.

Starting material: 4.1 g of 1-t-butyl-4-methoxybenezene;

Yield: 3.6 g;

NMR: δ = 6.7 - 7.7(3H,m), 3.8(3H,s), 3,1(2H,t), 2.2 - 2.6 (2H,m), 2.1(2H,d), 1.3(9H,s);

Mass: m/e = 278($M^+$), 263, 191.

Reference Example 4.

5-Chloro-2-hydroxy-4'-methoxycarbonylbenzophenone.

To 2 g of 5-Chloro-2-methoxy-4'-methoxycarbonylbenzophenone (prepared in Reference Example 3 (a).) was added 30 ml of 47% hydrobromic acid, and refluxed for 6.5 hours. The reaction solution was poured into ice-water, extracted with ethyl acetate, the extract was washed with a saturated aqueous solution of sodium chloride, dried over anhydrous sodium sulfate, and concentrated under reduced pressure. Obtained crystals by this procedure was washed with cyclohexane and dried. Obtained crystals were dissolved in 20 ml of acetone, added 869 mg of potassium carbonate and 0.8 ml of methyl iodide, and refluxed for 4 hours. After concentration, to the solution was added a saturated aqueous solution of ammonium chloride, extracted with ethyl acetate. The extract was washed with a saturated solution of sodium chloride, dried over anhydrous magnesium sulfate, and concentrated under reduced pressure. The residue was purified by column chromatography on silica gel using a mixture of cyclohexane and ethyl acetate (9:1) as an eluent to give 221 mg of the title compound having the following physical characteristics:

TLC (Cyclohexane : ethyl acetate = 5:1) : Rf=0.38;

IR : ν=1710, 1620, 1460, 1275, 1230, 1100, 950 $cm^{-1}$;

NMR: δ=11.80(1H, s), 8.22(2H, d), 7.74(2H, d),
7.44-7.6(2H, m), 7.06(1H, d).

By the procedure as described in Reference Example 4. the following compound. was given:

(a) 4-t-Butyl-2-(4-carboxylbutanoyl)phenol.

Starting material: 3.6 g of 1-t-butyl-4-methoxybenzene (prepared in Reference Example 3(b). );

Yield: 3.4 g;

NMR: δ =11.9(1H,s), 6.7 - 7.7(3H,m), 3.06(2H,t), 2.4(2H,d), 2.1(2H,q), 1.3(9H,s);

Mass: m/e = 264($M^+$), 249, 231, 177.

Reference Example 5.

5,4'-Dichloro-2-hydroxy-3-nitrobenzophenone.

1.4 g of the benzophenone compound (prepared in Reference Example 2.) was dissolved in 5 ml of acetic acid with heating, allowed to cool to room temperature, added one drop of concencrated sulfric acide, moreover, added dropwise 330 mg of concentrated nitric acid (60%) over a period of 20 minutes. After 5 minutes' stirring, the reaction mixture was cooled with ice, added a mixture of methylene chloride and ice-water, and extracted with methylene chloride. The extract was washed with a saturated aqueous solution of sodium chloride, dried over anhydrous sodium sulfate, and concentrated under reduced pressure. The residue was purified by column chromatography on silica gel using a mixture of methylene chloride

and n-hexane (1:1) as an eluent to give 1.5 g of crystals of the title compound having the following physical characteristics:

TLC(Methylene chloride): Rf=0.5;

NMR: δ=11.00(1H,s), 8.17(1H,d), 7.63(2H,d), 7.62(1H,d), 7.43(2H,d);

IR : ν=3420,3180, 3090, 1655, 1615, 1585, 1530, 1480, 1445 1405, 1315, 1280, 1245, 1160, 1090, 1010, 980, 905, 890, 845, 830, 780, 730 $cm^{-1}$;

Mass: m/e = 311($M^+$), 294, 276, 264, 199, 139, 111.

By the procedure as described in Reference Example 5, the following compounds were given:

(a) 4-Chloro-6-heptanoyl-2-nitrophenol.

(Instead of the phenol compound using in Reference Example 5, 1.28 g of the phenol compound prepared in Reference Example 2(b) was used.)

Yield: 1.4 g;

NMR: δ=11.5(1H,s), 8.05(1H,d), 7.7(1H,d), 3.0(2H,t);

Mass: m/e = 287, 285($M^+$)

(b) 5-Chloro-2-hydroxy-3-nitrobenzophenone.

(Instead of the phenol compound using in Reference Example 5, 1.23 g of 5-chloro-2-hydroxybenzophenone (commercial product by WAKO PURE CHEMICAL IND.) was used.)

Yield: 1.3 g;

Melting point: 56∼58 °C;

NMR: δ=11.23(1H,s), 8.13(1H,d), 7.90∼7.15(6H,m);

IR: $\nu$=3600-3515, 3075, 1665, 1630, 1605, 1590, 1525, 1440, 1395, 1310, 1270, 1240, 1160, 975, 895, 805, 765, 720, 695, 645 $cm^{-1}$;

Mass: m/e = 279, 278, 277($M^+$), 276, 267, 260, 105, 77.

Reference Example 6.

4'-Chloro-5-fluoro-2-hydroxy-3-nitrophenol.

3 g of the benzophenone compound (prepared in Reference Example 2(a).) was dissolved into 10 ml of acetic acid with heating and 1.3 g of concentrated nitric acid (60%) was added to the solution dropwise slowly over a period of 20 minutes. After 30 minutes' stirring at room temperature, a mixture of methylene chloride and ice-water was added to the solution with cooling with ice, the solution was extracted with methylene chloride. The extract was washed with a saturated aqueous solution of sodium chloride, concentrated under reduced pressure. The residue was purified by column chromatography on silica gel using a mixture of methylene chloride and n-hexane (1:1) as an eluent to give 2.99 g of yellow crystals of the title compound having the following physical characteristics:

TLC (Benzene): Rf= 0.58;

NMR: $\delta$=10.8(1H,s), 7.90(1H,dd), 7.63(2H,d), 7.40(2H,d), 7.7$\sim$7.2(1H,m);

IR : $\nu$=3450, 3225, 3100, 1655, 1600, 1585, 1570, 1540, 1485, 1455, 1425, 1350, 1310, 1290, 1250, 1220, 1155, 1095, 1005, 955, 885, 850, 800, 775, 755 $cm^{-1}$;

Mass: m/e = 295($M^+$), 260, 247, 184, 139, 111.

By the procedure as described in Reference Example 6, the following compounds were given:

(a) 5-Chloro-2-hydroxy-4'-methoxy-3-nitrobenzophenone.

(Instead of the benzophenone compound using in Reference Example 5, 1.3 g of the benzophenone compound prepared in Reference Example 3 was used, in addition, in the course of reaction 10 ml of methylene chloride was added.)

Yield: 1.2g;

TLC (Benzene): Rf=0.22;

NMR: δ=11.23(1H,s), 8.30∿8.15(1H,m), 7.90∿7.60(3H,m), 7.1∿6.9(2H,m), 3,90(3H,m);

IR : ν=3200, 3100, 1640, 1600, 1525, 1415, 1400, 1285, 1250, 1160, 1020, 975, 840 $cm^{-1}$;

Mass: m/e = 307($M^+$).

(b) 5-Chloro-2-hydroxy-4'-methoxycarbonyl-3-nitrobenzophenone.

Starting material: 409 mg of 5-Chloro-2-hydroxy-4'-methoxycarbonylbenzophenone (prepared in Reference Example 4.)

Yield: 412 mg;

Melting point: 140 - 145 °C;

IR : ν=1710, 1660, 1525, 1400, 1280, 1240, 1100, 980 $cm^{-1}$;

NMR: δ=8.30(1H, d), 8.19(2H, d), 7.86(2H, d), 7.76(1H, d), 3.98(3H, s);

Mass: 335($M^+$), 320, 304, 276, 163.

(c) 2-Hydroxy-4'-methoxy-3-nitro-5-phenylthiobenzophenone.

Starting material: 1.1 g of 2-hydroxy-4'-methoxy-5-phenylthiobenzophenone (prepared in Reference Example 2(c));

Yield: 463 mg;

NMR: δ = 11.43(1H,s), 8.21(1H,d), 7.76(2H,d), 7.70(1H,d), 7.3-(5H,s), 6.96(2H,d);

IR: ν = 3450, 3250, 0370, 2990, 2930, 2840, 1655, 1600, 1575, 1530, 1450, ;425, 1395, 1305, 1285, 1250, 1165, 1100, 1020, 980, 900, 845, 785, 740, 690, 645, 615 $cm^{-1}$;

Mass: m/e = 381(M ), 352, 334, 273, 243, 227, 286, 171, 135, 109, 92.

Reference Example 7.

5-t-Butyl-4'-chloro-2-hydroxy-3-nitrobenzophenone.

A solution of 1.8 g of 5-t-butyl-4'-chloro-2-hydroxybenzophenone (prepared in a similar manner as described in Reference Example 1-3 of the specification of our British Patent Publication No. 2026480.) solved in 2 ml of methylene chloride was dropped to 2 ml of concentrated sulfuric acid cooled to 0°C over a period of 2 minutes with stirring. After that, a mixture of one ml of concentrated sulfuric acid and 0.61 ml of concentrated nitric acid was added dropwise to the solution over a period of 5 minutes at 5 °C. After 30 minutes' stirring, the reaction mixture was poured into 50 ml of ice-water, and extracted with ethylether. Ether layer was washed with water, an aqueous solution of sodium hydrocarbonate, water successively, dried over anhydrous magnesium sulfate,

and concentrated under reduced pressure. The residue was purified by column chromatography on silica gel using a mixture of ethyl acetate and cyclohexane (1:8) as an eluent to give in a 43.1% yield, 906 mg of the title compound having the following physical characteristics:

Melting point: 96 - 98 °C;

TLC (Ethyl acetate : cyclohexane = 1:5) : Rf=0.44;

NMR: δ=10.6(1H, s), 8.10(1H, d), 7.5(5H, m), 1.30(9H, s).

By the procedure as described in Reference Example 7, the following compounds were given:

(a) 2-Propionyl-4-t-butyl-6-nitrophenol.

Starting material: 2.0 g of 2-propionyl-4-t-butylphenol (the compound described in Reference Example 3(c) of the specification of our British Patent Publication No. 2026480.);

Yield: 1.2 g;

NMR: δ = 8.07(1H,d), 7.95(1H,d), 3.09(2H,q), 1.34(9H,s), 1.23(3H,t).

(b) 2-Benzoyl-4-tert-butyl-6-nitrophenol.

Starting material: 2.406 g of 2-Benzoyl-4-tert-butylphenol (the compound described in Reference Example 3(a) of the specification of our British Patent Publicarion No.2026480);

Yield: 1.206 g;

NMR ($CCl_4$ solution): δ = 10.5(1H,s), 8.05(1H,d), 7.5(6H,m), 1.33(9H,s);

Mass: m/e = 299 ($M^+$), 284, 269, 254, 206.

(c) 2-Heptanoyl-4-tert-butyl-6-nitrophenol.

Starting material: 1.672 g of 2-Heptanoyl-4-tert-butylphenol (prepared in a similar manner as described in Reference Example 1-3 of the specification of our British Patent Publication No.2026480.);

Yield: 1.278 g;

NMR ($CCl_4$ solution): δ = 12.2(1H,s), 7.95(1H,d), 7.85(1H,d), 3.0(2H,t), 1.9-0.7(10H,m).

(d) 4-t-Butyl-2-(4-carboxybutanoyl)-6-nitrophenol.

Starting material: 3.4 g of 4-t-butyl-2-(4-carboxybutanoyl)-phenol (prepared in Reference Example 4(a));

Yield: 1.85 g;

NMR: δ = 12.3(1H,s), 8.1(2H,m), 3.06(2H,t), 2.4(2H,d), 2.1 (2H,q), 1.3(9H,s);

Mass: m/e = 309 ($M^+$), 291, 222.

Reference Example 8.

4-t-Butyl-2-(4-ethoxycarbonylbutanoyl)-6-nitrophenol.

547 mg of 4-t-butyl-2-(4-carboxybutanoyl)-6-nitrophenol (prepared in Reference Example 7(d).) dissolved in 25 ml of a mixture of ethanol and hydrochloride, the solution was refluxed for 12 hours, and extracted with ethyl acetate. The extract was washed with water, a saturated aqueous solution of sodium carbonate, water, a saturated aqueous solution of sodium chloride, succesively, dried over anhydrous magnesium sulfate, and concentrated

under reduced pressure. The residue was purified by column chromatography using a mixture of cyclohexane and ethyl acetate (5:1) as an eluent to give 190 mg of the title compound having the following physical characteristics:

NMR: $\delta$ = 12.2(1H,s), 7.9(2H,s), 4.1(2H,q), 2.9 - 3.4(2H,m), 1.9 - 2.7(4H,m), 1.4(9H,s), 1.2(3H,t);

Mass: m/e = 337($M^+$), 319, 222.

Example 1.

3-Amino-5-chloro-2-hydroxy-4'- methoxybenzophenone hydrochloride.

To the suspension of 165 mg of the benzophenone compound (prepared in Reference Example 6 (a).) in 4 ml of water was added 2 ml of concentrated aqueous ammonia, 1.4 g of sodium hydrosulfide dissolved in 6 ml of water all at once, tetrahydrofuran to solve solids, and stirred for 15 minutes. The reaction mixture was extracted with chloroform, the extract was washed with a saturated aqueous solution of sodium chloride, dried over anhydrous sodium sulfate, and concentrated under reduced pressure. The residue was dissolved in ethanol, after addition of 0.2 ml of concentrated hydrochloric acid, the solution was concentrated. The residue was dissolved in ethanol again, the solution was treated with active carbon, concentrated under reduced pressure, and dried to give 106 mg of the title compound having the following physical characteristics:

Melting point: 125 - 135 °C;

TLC (Methylene chloride): Rf=0.52;

IR : $\nu$=3420, 3320, 2770, 2580, 1630, 1590, 1555, 1440, 1305, 1250, 1160, 840, 615 $cm^{-1}$;

NMR (Dimethylsulfoxide-$d_6$ solution):

$\delta$=8.0-7.6(3H, m), 7.3-7.0(3H, m), 3.92(3H, s);

Mass: m/e = 277($M^+$), 169.

By the procedure as described in Example 1. the following compounds were given:

(a) 3-Amino-5-chloro-2-hydroxy-4'-methoxycarbonylbenzophenone hydrochloride.

Starting material: 100 mg of 5-Chloro-2-hydroxy-4'-methoxycarbonyl-3-nitrobenzophenone (prepared in Reference Example 6(b).);

Yield: 65 mg;

Melting point: 110 - 115 °C;

TLC (Cyclohexane : ethyl acetate = 5 : 1): Rf=0.26;

IR : $\nu$=1725, 1640, 1455, 1285, 1110, 1010, 770 $cm^{-1}$;

NMR: $\delta$=8.21(2H, d), 7.85(2H, d), 7.71(1H, d), 7.52(1H, d), 3.97(3H, s);

Mass: m/e = 305($M^+$).

(b) 3-Amino-2-hydroxy-4'-methoxy-5-phenylthiobenzophenone hydrochloride.

Starting material: 463 mg of 2-hydroxy-4'-methoxy-3-nitro-5-phenylthiobenzophenone ( prepared in Reference Example 6(c) )

Yield: 119 mg;

Melting point: 95 - 98°C;

TLC( Ethyl acetate : benzene = 1;10): Rf = 0.45;

NMR( $CDCl_3$ + $CD_3OD$ ): δ = 7.65(2H,d), 7.63(2H,s), 7.73(5H,s), 6.98(2H,d), 3.93(3H,s);

IR: ν = 3450, 3050, 2840, 2550, 1635, 1600, 1565, 1510, 1450, 1330, 1310, 1260, 1170, 1015, 1000, 845, 790 $cm^{-1}$;

Mass: m/e = 351, 243, 215, 286, 135, 110.

Example 2.

3-Amino-5,4'-dichloro-2-hydroxybenzophenone.

370 mg of the nitrobenzophenone compound (prepared in Reference Example 7.) was dissolved in 10 ml of ethanol, one ml of chloroform and 50 mg of palladiumcarbon (10% content) were added to the solution, and hydrogen gas was bubbled into the mixture for 2 hours with stirring. After reaction, catalyst was removed by filtration, the filtrate was concentrated under reduced pressure. The residue was purified by column chromatography on silica gel using methylene chloride as an eluent to give 70 mg of crystals of the title compound having the following physical characteristics:

Melting point: 91 - 94 °C (Decomposed.);

TLC (methylene chloride) : Rf=0.36;

IR : ν=3460, 3320, 3050, 2925, 1620, 1600, 1560, 1460, 1395, 1365, 1330, 1315, 1275, 1225, 1195, 1180, 1095, 1040,

1020, 920, 855, 845, 820, 785, 765, 735, 715 $cm^{-1}$;

NMR: δ=12.0(1H, br-s), 7.62(2H, d), 7.50(2H, d), 6.87(2H, s), 3.84(2H, br-s);

Mass: m/e = 283, 281($M^+$), 171, 169, 141, 139, 113, 111.

By the procedure as described in Example 2, the following compounds was given:

(a) 3-Amino-4'-chloro-5-fluoro-2-hydroxybenzophenone.

Starting material: 500 mg of 4'-Chloro-5-fluoro-2-hydroxy -3-nitrobenzophenone (prepared in Reference Example 6.);

Yield: 400 mg;

Melting point: 124 - 127 °C;

TLC (Methylene chloride): Rf=0.48;

IR : ν=3480, 3340, 3060, 2930, 1630, 1600, 1590, 1560, 1475, 1440, 1400, 1385, 1335, 1280, 1220, 1195, 1180, 1120, 1095, 1045, 1015, 1000, 855, 845, 835, 785 $cm^{-1}$;

NMR: δ=11.85(1H, br-s), 7.62(2H, d), 7.48(2H, d), 6.75(1H, dd), 6.56(1H, dd), 4.03(2H, br-s);

Mass: m/e = 267, 265($M^+$), 153, 139, 125, 111, 97.

Example 3.

3-Amino-5-chloro-2-hydroxybenzophenone hydrochloride.

By the procedure as described in Example 2, using 233 mg of the nitrobenzophenone compound(prepared in Reference Example 5(b).), crystals of 3-amino-5-chloro-2-hydroxybenzophenone were given. Obtained crystals were dissolved in 10 ml of ethanol, 0.42 ml of 2N hydrochloric acid was dropped to the solution at room temperature. The reaction mixture was concentrated to give 65 mg of the title compound having the following physical characteristics:

Melting point: 93 - 95 °C;

TLC (Chloroform : ethanol = 19:1): Rf=0.80;

NMR (Deuterochloroform + acetone-$d_6$ solution):

$\delta$=7.84~7.37(5H, m), 6.91(1H, d), 6.86(1H, d);

IR: $\nu$=3470, 3330, 1615, 1590, 1455, 1355, 1305, 1270, 1220, 1185, 1170, 1015, 905, 855, 795, 765, 695, 660 $cm^{-1}$;

Mass: m/e = 249, 247, 170, 168, 148, 112, 104, 77, 76.

By the procedure as described in Example 3, the following compounds were given:

(a) 2-Amino-4-chloro-6-heptanoylphenol hydrochloride.

(Instead of the nitrobenzophenone compound using in Example 3, 240 mg of the nitrophenol compound prepared in Reference

Example 5(a) was used.)

Yield: 62 mg;

NMR: δ=12.2(1H, s), 10.0(3H, broad-s), 7.9(1H, d), 7.7(1H, d), 2.9(2H, t);

IR : ν=3400, 3300-2650, 1650, 1620, 1570 $cm^{-1}$;

Mass: m/e = 302, 300.

(b) 2-Amino-4-t-butyl-6-(4-ethoxycarbonylbutyryl)phenol hydrochloride.

Starting material: 212 mg of 4-t-butyl-6-(4-ethoxycarbonylbutyryl)-2-nitrophenol ( prepared in Reference Example 8.);

Yield: 136 mg;

TLC( Methylene chloride ; ethyl acetate = 50:1 ): Rf = 0.27;

NMR( $CCl_4$ ): δ = 10.1(4H,b), 8.0(1H,s), 7.6(1H,s), 4.0(2H,q), 3.0(2H,m), 2.35(2H,t), 2.0 - 1.2(12H,m);

IR: ν = 3400, 3300 - 2300, 1720, 1650, 1470, 1360, 1340, 1270, 1200, 1090, 1050 $cm^{-1}$;

Mass: m/e = 307, 292, 279, 262, 261, 245, 233, 219, 204, 192, 176.

Example 4.

3-Amino-4-t-butyl-4'-chloro-2-hydroxybenzophenone hydrochloride.

800 mg of 4-t-Butyl-4'-chloro-2-hydroxy-3-nitrobenzophenone (prepared in Reference Example 6.) was dissolved in a mixed solution of 10 ml of benzene and 4 ml of tetrahydrofuran,

the solution was added 13 ml of an aqueous solution of titanium trichloride, and stirred for 20 hours at room temperature. The reaction mixture was poured into 100 ml of water added 15 ml of concentrated aqueous ammonia, extracted with methylene chloride. The layer of methylene chloride was washed with water, dried over anhydrous sodium sulfate, and concentrated under reduced pressure. The residue (red oil) was purified by column chromatography on silica gel using a mixture of methylene chloride and cyclohexane (1:8) as an eluent, and fractions containing purpose compound were collected, added concentrated hydrochloric acid to make acidic, and concentrated. Obtained crystals were washed with a mixture of methylene chloride and cyclohexane (1:15), filtrated, and dried to give 425 mg of yellow crystals of the title compound having the following physical characteristics:

Yield: 52.1%;

Melting point: 153 - 158 °C;

TLC (Ethyl acetate : cyclohexane = 1:4) : Rf=0.42 (value of free amine);

IR : ν=3400, 3200-2650, 2550, 1640, 1600, 1550, 1460, 1340, 1310 $cm^{-1}$;

NMR: δ=11.0(3H, br-s), 8.0(1H, d), 7.4(5H, m), 1.20(9H, s);

Mass: m/e = 303($M^+$), 298, 191, 177, 176, 141, 139;

Elementary analysis:

| | C(%) | H(%) | N(%) |
|---|---|---|---|
| calcd. | 60.01 | 5.65 | 4.12 |
| found | 60.23 | 5.48 | 4.30 |

By the procedure as described in Example 4, the following compounds were given:

(a) 2-Amino-4-t-butyl-6-heptanoylphenol hydrochloride.

Starting material: 1.28 g of 2-Heptanoyl-4-t-butyl-6-nitrophenol (prepared in Reference Example 6(a).);

Yield: 680 mg;

Melting point: 125 - 126 °C;

TLC (Ethyl acetate : cyclohexane = 1:8) : Rf = 0.36 (value of free amine);

IR : ν=3400, 3300-2650, 2550, 1950, 1650, 1620, 1570, 1520, 1450, 1280 $cm^{-1}$;

NMR (Deuterochloroform + carbon tetrachloride):

δ=12.5(1H, b-s), 10.2(2H, br-s), 7.95(1H, d), 7.60(1H, d), 2.9(2H, t), 1.9-0.6(10H, m);

Mass: m/e = 277($M^+$), 261, 244, 234, 192, 164;

Elementary analysis:

| | C(%) | H(%) | N(%) |
|---|---|---|---|
| calcd. | 65.06 | 8.99 | 4.46 |
| found | 65.29 | 8.71 | 4.41 |

(b) 3-Amino-5-t-butyl-2-hydroxybenzophenone hydrochloride.

Starting material: 1.2 g of the nitro compound prepared in Reference Example 7 (b);

Yield: 418 mg;

Melting point: 123 - 124°C;

TLC( Ethyl acetate:cyclohexane=1:4 ): Rf=0.43(as free amine);

NMR: δ = 9.3(3H,b-s), 8.0(1H,d), 7.5(6H,m), 1.20(9H,s);

IR: ν = 3500, 3300, 3250-2200, 1650, 1590, 1570, 1460, 1340 $cm^{-1}$;

MS: m/e = 269($M^+$), 254, 191, 176, 105;

Elementary analysis:

| | C | H | N |
|---|---|---|---|
| calcd. | 66.77 | 6.59 | 4.58 (%) |
| found | 66.51 | 6.62 | 4.34 (%) |

Example 5.

2-Amino-4-t-butyl-6-propionylphenol.

To 1.025 g of 2-propionyl-4-tert-butyl-6-nitrophenol (prepared in Reference Example 7(a).) dissolved in 0.5 ml of benzene was added 18.6 ml of a 20% aqueous solution of titanium trichloride. After 10 minutes' shaking, the mixture was stirred vigorously for 24 hours. A saturated aqueous solution of sodium hydrogencarbonate and chloroform were added to the reaction mixture and the mixture was extracted with chloroform three times. The organic layers were combined and washed with water, dehydrated and concentrated under reduced pressure. The residue was purified by column chromatography on silica gel using a mixture of ethyl acetate and benzene (1:3) as an eluent to give 848 mg of the title compound having the following physical characteristics:

NMR: δ = 7.06(1H,d), 6.87(1H,d), 2.97(2H,q), 1.28(9H,s), 1.18(3H,t);

Mass: m/e = 221($M^+$), 206.

Example 6.

2-Amino-4-t-butyl-6-propionylphenol hydrochloride.

To 848 mg of 2-propionyl-4-tert-butyl-6-aminophenol

(prepared in Example 5) dissolved in 1 ml of ethanol was added an equivalent ethanol-hydrochloric acid solution and concentrated under reduced pressure. The obtained crystals were dissolved in methylene chloride and crystallized by addition of n-hexane. The resulting crystals were separated from the solution by filtration to give 880 mg of the title compound having the following physical characteristics:

Melting point: 130 - 137°C (Decomposed);

TLC( n-Butanol: acetic acid:water=10:1:1 ): Rf=0.57;

NMR: δ = 9.6(3H,b-s), 8.05(1H,d), 7.76(1H,d), 3.05(2H,q), 1.32(9H,s), 1.23(3H,t);

IR: ν = 3400, 2900, 1655, 1475, 1370, 1295, 1230, 1110, 820 $cm^{-1}$;

Mass: m/e = 221, 206, 192, 188;

Elementary analysis:

| | C | H | N |
|---|---|---|---|
| calcd. | 60.58 | 7.82 | 5.43 (%) |
| found | 60.75 | 8.10 | 5.21 (%) |

Example 7.

3-Amino-5-t-butyl-4'-chloro-2-hydroxybenzhydrol hydrochloride.

In an atmosphere of nitrogen, to 6 ml of the solution of 194 mg of 3-amino-5-t-butyl-4'-chloro-2-hydroxybenzophenone hydrochloride (prepared in Example 4.) dissolved in methanol was added slowly 44 mg of sodium borohydride at 0 °C, and stirred gently. The mixture was extracted with ethyl acetate, the extract

was washed with a saturated solution of sodium chloride, dried over anhydrous sodium sulfate, and concentrated under reduced pressure. The residue was dissolved in methylene chloride, filtered off impurities, the filtrate was concentrated. The residue was dissolved in methylene chloride, added 10 ml of a mixture of ethanol and hydrochloric acid (100:1), concentrated under reduced pressure, added chloroform, and concentrated under reduced pressure. Deposited crystals were washed with ethyl acetate, filtered, and dried under reduced pressure to give 128 mg of the title compound having the following physical characteristics in a 66% yield:

Melting point: 183 - 186 °C

TLC (Methylene chloride : methanol : acetic acid = 100:5:1): Rf=0.44;

IR : ν=3400, 3250, 2960, 2600, 1635, 1485, 1395, 1305, 1195, 1090, 1010, 850, 820, 780 $cm^{-1}$;

NMR (Deuterochloroform + dimethylsulfoxide-$d_6$ solution); δ=7.42(1H, d), 7.37(2H, d), 7.26(2H, d), 7.15(1H, d), 6.07(1H, s), 1.23(9H, s);

Mass: m/e = 305($M^+$), 287, 272, 252, 149.

By the procedure as described in Example 7, the following compound were given:

(a) 3-Amino-4'-chloro-5-fluoro-2-hydroxybenzhydrol hydrochloride.

Starting material: 408 mg of 3-Amino-4'-chloro-5-fluoro-2-hydroxybenzophenone (prepared in Example 2(a).);

Yield: 329 mg (80%);

Melting point: 155 - 180 °C (Decomposed);

TLC (Chloroform : methanol : aqueous ammonia = 60:6:1): Rf=0.41;

IR : ν=3400, 2570, 1630, 1470, 1215, 1185, 1090, 1010, 960, 840, 760, 675, 615 $cm^{-1}$;

NMR (Dimethylsulfoxide-$d_6$ solution):

δ=7.40(4H, s), 7.12(1H, s), 7.04(1H, s), 6.15(1H, s);

Mass: m/e = 267($M^+$, 11), 249(50), 214(100).

(b) 3-Amino-5,4'-dichloro-2-hydroxybenzhydrol hydrochloride.

Starting material: 408 mg of 3-Amino-5,4'-dichloro-2-hydroxybenzophenone (prepared in Example 2.);

Yield: 317 mg (77.5%);

Melting point: 145 - 160 °C;

TLC (Chloroform : methanol : aqueous ammonia = 60:6:1): Rf=0.35;

IR : ν=3400, 3250, 2850, 2600, 1940, 1625, 1465, 1280, 1260, 1200, 1085, 1005, 845, 660, 600, 530 $cm^{-1}$;

NMR (Dimethylsulfoxide-$d_6$ solution):

δ=7.40(4H, s), 7.25-7.45(2H, m), 6.2(1H, s);

Mass: m/e = 283($M^+$, 11), 267(68), 232(36), 230(100).

(c) 3-Amino-5-chloro-2-hydroxy-4'-methoxycarbonylbenzhydrol hydrochloride.

Starting material: 45 mg of 3-Amino-5-chloro-2-hydroxy-4'-methoxycarbonylbenzophenone hydrochloride (prepared in Example 1(a).);

Yield: 28 mg;

Melting point: 160 - 170 °C (Decomposed.);

TLC (Chloroform : methanol = 10 : 1): Rf=0.45;

IR : ν=2860, 2600, 1720, 1470, 1280 $cm^{-1}$;

NMR (Methanol-$d_4$):

δ=8.01(2H, d), 7.54(2H, d), 7.23(1H, d), 7.20(1H, d), 6.12(1H, s), 3.89(3H, s).

(d) 3-Amino-2-hydroxy-4'-methoxy-5-phenylthiobenzhydrol hydrochloride.

Starting material: 243 mg of 3-amino-2-hydroxy-4'-methoxy-5-phenylthiobenzophenone (prepared in Example 1(b).):

Yield: 194 mg;

Melting point: more than 120°C (decomposed);

TLC( Ethyl acetate : benzene = 1:10 ): Rf = 0.42;

NMR($CDCl_3$ + $CD_3OD$ ): δ = 7.35 - 7.15(8H,m), 7.00(1H,d), 6.88 (2H,d), 5.64(1H,s), 3.83(3H,s);

IR: ν = 3450, 3050, 2980, 2840, 2570, 1610, 1580,1510, 1470, 1250, 1170, 1060, 1030, 835, 740, 690 $cm^{-1}$;

Mass: m/e = 353, 335, 321, 305, 243, 189, 175, 119, 110.

Example 8.

2-Amino-4-t-butyl-6-(1-hydroxyheptyl)phenol.

In an atmosphere of nitrogen, to 3 ml of a solution of 100 mg of 6-amino-4-t-butyl-2-heptanoylphenol hydrochloride in methanol was added 36 mg of sodium borohydrride in limited amounts

(prepared in Example 4(a).) in methanol was added 36 mg of sodium borohydride in limited amounts at 0°C, and stirred for 15 minutes. The mixture was extracted with ethyl acetate, the extract was washed with a saturated aqueous solution of sodium chloride, dried over anhydrous sodium sulfate, and concentrated under reduced pressure. The residue was purified by column chromatography on silica gel using a mixture of chloroform and methanol (20:1) as an eluent to give the title compound having the following physical characteristics in a 76% yield:

Melting point: 85 °C;

TLC (Chloroform : methanol : aqueous ammonia = 60 : 6 : 1): Rf=0.60;

IR : ν=3425, 3320, 3100, 2955, 2930, 2850, 1605, 1495, 1250, 1220, 855, 740 $cm^{-1}$;

NMR: δ=6.5-6.75(1H, m), 6.2-6.4(1H, m), 4.6-4.8(1H, m), 1.65-2.0(2H, m), 1.1-1.5(8H, m), 1.23(9H, s), 0.75-1.0(3H, m);

Mass: m/e = 279($M^+$, 21), 261(100), 246(54).

By the procedure as described in Example 8, the following compounds were given:

(a) 3-Amino-5-chloro-2-hydroxy-4'-methoxybenzhydrol.

Starting material: 308 g of 3-Amino-5-chloro-2-hydroxy-4'-methoxybenzophenone hydrochloride (prepared in Example 1.);

Yield: 164 mg (60%);

TLC (Chloroform : methanol = 20 : 1): Rf=0.50;

IR (Chloroform solution):

ν=3350, 1605, 1505, 1480, 1250, 1175, 1030, 835 $cm^{-1}$;

NMR: δ=7.1-7.4(2H, m), 6.7-7.0(2H, m), 6.56(1H, d), 6.21(1H, d), 5.75(1H, s), 3.77(3H, s);

Example 9.

3-Amino-4'-chloro-5-fluoro-2-hydroxybenzophenoneoxime hydrochloride.

To 900 mg of the benzophenone compound (prepared in Example 2(a).) dissolved in 50 ml of ethanol was added one g of hydroxyamine hydrochloride and one g of sodium carbonate, and the solution was refluxed for 6 hours. After reaction, the solution was allowed to cool, removed ethanol, added chloroform and water, and extracted with chloroform. The extract was washed with a saturated aqueous solution of sodium chloride, dried over anhydrous sodium sulfate, and concentrated under reduced pressure to give 794 mg of crude crystals. Obtained product was dissolved in ethanol, 0.24 ml of concentrated hydrochloric acid was added to the solution, and dried under reduced pressure. Obtained hydrochloride was dissolved in ethanol, treated with active carbon, and recrystallized from a mixture of ethanol and diethylether (1:2) to give 163 mg of the title compound having the following physical characteristics:

Melting point: 183 - 191 °C (Decomposed.);

TLC (Methylene chloride : methanol = 10:1): Rf= 0.53;

NMR (Methanol-$d_4$ solution):

δ=7.56(2H, d), 7.33(2H, d), 7.26(1H, dd), 6.64(1H, dd);

IR: ν=3400, 3250, 3030, 2900(broad), 2580, 1590, 1550, 1470, 1410, 1395, 1340, 1285, 1225, 1200, 1130, 1095, 1050, 1015, 995, 880, 840, 805, 795, 750 $cm^{-1}$;

Mass: m/e = 280($M^+$), 262, 151, 139, 126, 123, 111, 97.

Example 10.

2-Amino-4-chloro-6-(α-ethoxy-4-methoxybenzyl)phenol hydrochloride.

In an atomosphere of nitrogen, 308 mg of 3-amino-5-chloro-2-hydroxy-4'-methoxybenzophenone hydrochloride (prepared in Example 1.) dissolved in 10 ml of methanol was added 76 mg of sodium borohydride at 0°C, and stirred for 15 minutes. The mixture was extracted with ethyl acetate, the extract was washed with a saturated aqueous solution of sodium chloride, dried and concentrated under reduced pressure. The residue was dissolved in methylene chloride, filtered and concentrated. The residue was dissolved in ethanol and to the solution, 0.2 ml of concentrated hydrochloric acid was added, concentrated. The residue was added chloroform and concentrated to give 100 mg of the title compound having the following physical characteristics:

Melting point: 150 - 160°C;

NMR( DMSO-$d_6$ ): δ = 7.2 - 7.4(4H,m), 6.8 - 7.0(2H,m), 5.91 (1H,b-s), 3.73(3H,s), 3.46(2H,q), 1.18 (3H,t);

IR: ν = 3350, 2850, 2580, 1605, 1505, 1460, 1280, 1255, 1170, 1070 $cm^{-1}$;

Mass: m/e = 307($M^+$), 261, 230.

Example 11.

5 g of 3-Amino-5,4'-dichloro-2-hydroxybenzophenone, 200 mg of cellulose magnesium gluconate (disintegrator), 100 mg of magnesium stearate (lubricating agent) and 4.7 g of crystal cellulose were mixed and punched out in a conventional method to obtain 100 tablets containing 50 mg of the active ingredient in one tablet.

Example 12.

5 g of 3-Amino-5-t-butyl-4'-chloro-2-hydroxybenzhydrol hydrochloride, 200 mg of cellulose magnesium gluconate (disintegrator), 100 mg of magnesium stearate (lubricating agent) and 4.7 g of crystal cellulose were mixed and punched out in a conventional method to obtain 100 tablets containing 50 mg of the active ingredient in one tablet.

CLAIMS for the Contracting States:-

BE - CH - DE- FR - GB - IT - LU - NL - SE

1. A 2-aminophenol derivative of the general formula:

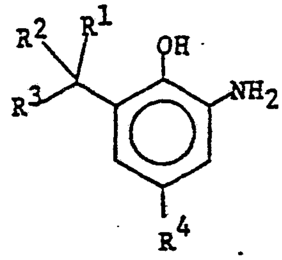

(I)

[wherein $R^1$ represents a straight or branched chain alkyl group containing from 1 to 6 carbon atom(s), a group represented by the general formula:

$$-(CH_2)n-COOR^5 \qquad (II)$$

(wherein $R^5$ represents a hydrogen atom or a straight or branched chain alkyl group containing from 1 to 4 carbon atom(s) and n represents zero or an integer from 1 to 6) or a group represented by the general formula:

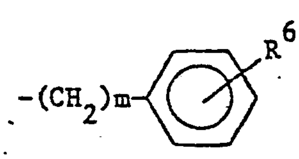

(III)

(wherein $R^6$ represents a hydrogen atom, a halogen atom, a straight or branched chain alkyl group containing from 1 to 4 carbon atom(s), a straight or branched chain alkoxyl group containing from 1 to 4 carbon atom(s), a carbamoyl group, an alkoxycarbonyl group containing from 2 to 6 carbon atoms, a carboxyl group, an amino group, a sulfamoyl group or a cyano group and m represents zero or an integer of 1 or 2.), $R^2$ represents a hydroxyl group, a straight or branched chain alkoxyl group containing from 1 to 4 carbon atom(s) or together with $R^3$ an oxo group or a hydroxyimino group, $R^3$ represents a hydrogen atom or together with $R^2$ an oxo group or a hydroxyimino group, and $R^4$ represents a halogen atom, a straight or branched chain alkyl group containing from 1 to 6 carbon atom(s) or a phenylthio group, but not including a compound wherein $R^2$ and $R^3$ taken together represent an oxo group, $R^1$ represents a methyl group and $R^4$ represents a methyl group];
or a pharmaceuitcal acceptable acid addition salt thereof.

2.A compound according to claim 1, wherein $R^2$ represents a hydroxyl group and $R^3$ represents a hydrogen atom.

3. A compound according to claim 1, wherein $R^2$ and $R^3$ taken together represent an oxo group.

4. A compound according to claim 1, wherein $R^2$ and $R^3$ together represent a hydroxyimino group.

5. A compound according to claim 1, wherein $R^2$ represents an alkoxyl group containing from 1 to 4 carbon atom(s) and $R^3$ represents a hydrogen atom.

6. A compound according to claim 2, wherein $R^1$ represents the group represented by the general formula (III).

7. A compound according to claim 3, wherein $R^1$ represents the group represented by the general formula (III) which is depicted in claim 1 wherein various symbols are as defined in claim 1.

8. A compound according to claim 4, wherein $R^1$ represents the group represented by the general formula (III).

9. A compound according to claim 5, wherein $R^1$ represents the group represented by the general formula (III).

10. A compound according to claim 2, wherein $R^1$ represents an alkyl group containing from 1 to 6 carbon atom(s).

11. A compound according to claim 3, wherein $R^1$ represents an alkyl group containing from 1 to 6 carbon atom(s) or the group represented by the general formula (II) which is depicted in claim 1 wherein various symbols are as defined in claim 1.

12. A compound according to claim 6, wherein $R^6$ represents a halogen atom and $R^4$ represents a straight or branched chain alkyl group containing from 1 to 6 carbon atom(s) or a halogen atom.

13. A compound according to claim 12, which is 3-amino-5,4'-dichloro-2-hydroxybenzhydrol or its hydrochloride.

14. A compound according to claim 12, which is 3-amino-4'-chloro-5-fluoro-2-hydroxybenzhydrol or its hydrochloride.

15. A compound according to claim 12, which is 3-amino-5-t-butyl-4'-chloro-2-hydroxybenzhydrol or its hydrochloride.

16. A compound according to claim 6, wherein $R^6$ represents a straight or branched chain alkoxyl group containing from 1 to 4 carbon atom(s) or an alkoxycarbonyl group containing from 2 to 6 carbon atoms and $R^4$ represents a halogen atom or a phenylthio group.

17. A compound according to claim 16, which is 3-amino-5-chloro-2-hydroxy-4'-methoxybenzhydrol or its hydrochloride.

18. A compound according to claim 16, which is 3-amino-5-chloro-2-hydroxy-4'-methoxycarbonylbenzhydrol or its hydrochloride.

19. A compound according to claim 16, which is 3-amino--2-hydroxy-4'-methoxy-5-phenylthiobenzhydrol or its hydrochloride.

20. A compound according to claim 10, wherein $R^4$ represents a straight or branched chain alkyl group containing from 1 to 6 carbon atom(s).

21. A compound according to claim 20, which is 2-amino-4-t-butyl-6-(1-hydroxyheptyl)phenol or its hydrochloride.

22. A compound according to claim 7, wherein $R^6$ represents a hydrogen atom and $R^4$ represents a halogen atom or a straight or branched chain alkyl group containing from 1 to 6 carbon atom(s).

23. A compound according to claim 22, which is 3-amino-5-t-butyl-2-hydroxybenzophenone or its hydrochloride.

24. A compound according to claim 22, which is 3-amino-5-chloro-2-hydroxybenzophenone or its hydrochloride.

25. A compound according to claim 7, wherein $R^6$ represents a halogen atom and $R^4$ represents a halogen atom or a straight or branched chain alkyl group containing from 1 to 6 carbon atom(s).

26. A compound according to claim 25, which is 3-amino-5,4'-dichloro-2-hydroxybenzophenone or its hydrochloride.

27. A compound according to claim 25, which is 3-amino-4'-chloro-5-fluoro-2-hydroxybenzophenone or its hydrochloride.

28. A compound according to claim 25, which is 3-amino-5-t-butyl-4'chloro-2-hydroxybenzophenone or its hydrochloride.

29. A compound according to claim 7, wherein $R^6$ represents a straight or branched chain alkoxyl group containing from 1 to 4 carbon atom(s) or an alkoxycarbonyl group containing from 2 to 6 carbon atoms and $R^4$ represents a halogen atom or a phenylthio group.

30. A compound according to claim 29, which is 3-amino-5-chloro-2-hydroxy-4'-methoxybenzophenone or its hydrochloride.

31. A compound according to claim 29, which is 3-amino-5-chloro-2-hydroxy-4'-methoxycarbonylbenzophenone or its hydrochloride.

32. A compound according to claim 29, which is 3-amino-2-hydroxy-4'-methoxy-5-phenylthiobenzophenone or its hydrochloride.

33. A compound according to claim 11, wherein $R^1$ represents a straight or branched chain alkyl group containing from 1 to 6 carbon atom(s) and $R^4$ represents a halogen atom or a straight or branched chain alkyl group containing from 1 to 6 carbon atom(s).

34. A compound according to claim 33 which is 2-amino-4-chloro-6-heptanoylphenol or its hydrochloride.

35. A compound according to claim 33 which is 2-amino-4-t-butyl-6-heptanoylphenol or its hydrochloride.

36. A compound according to claim 33 which is 2-amino-4-t-butyl-6-propionylphenol or its hydrochloride.

37. A compound according to claim 11, wherein $R^1$ represents the group represented by the general formual (II) and $R^4$ represents a straight or branched chain alkyl group containing from 1 to 6 carbon atom(s).

38. A compound according to claim 37 which is 2-amino-4-t-butyl-6-(4-ethoxycarbonylbutyryl)phenol or its hydrochloride.

39. A compound according to claim 8, wherein $R^6$ represents a halogen atom and $R^4$ represents a halogen atom.

40. A compound according to claim 39 which is 3-amino-4'-chloro-5-fluoro-2-hydroxybenzophenoneoxime or its hydrochloride.

41. A compound according to claim 9, wherein $R^6$ represents a straight or branched chain alkoxyl group containing from 1 to 4 carbon atom(s) and $R^4$ represents a halogen atom.

42. A compound according to claim 41 which is 2-amino-4-chloro-6-(α-ethoxy-4-methoxybenzyl)phenol or its hydrochloride.

43. A method for the preparation of a 2-aminophenol derivative of the general formula (I) or a pharmaceutically acceptable acid addition salt thereof:

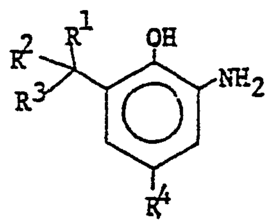

(I)

[wherein $R^1$, $R^2$, $R^3$ and $R^4$ are as defined in claim 1]
which comprises selectively reducing the nitro group of a compound of the general formula:

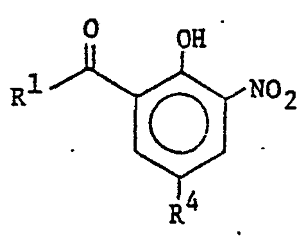

(IV)

[wherein $R^1$ and $R^4$ are as defined in claim 1] to an amino group to obtain a compound of the general formula:

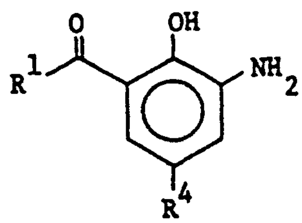

(IB)

[wherein $R^1$ and $R^4$ are as defined in claim 1], and, if desired to obtain a compound of the general formula:

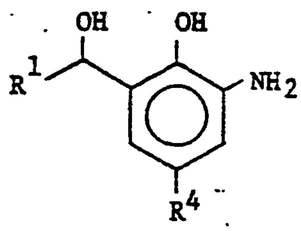

(IA)

[wherein $R^1$ and $R^4$ are as defined in claim 1] selectively reducing the oxo group of a compound of the general formula (IB) to a hydroxyl group, and further, if desired to obtain a compound of the general formula:

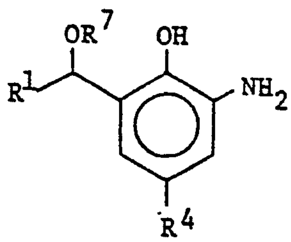

(ID)

[wherein $R^7$ represents a straight or branched chain alkyl group containing from 1 to 4 carbon atom(s) and $R^1$ and $R^4$ are as defined in claim 1],
converting the hydroxyl group at the α-position of a compound of the general formula (IA) to an alkoxyl group represented by-$OR^7$; or if desired to obtain compounds of general formula:

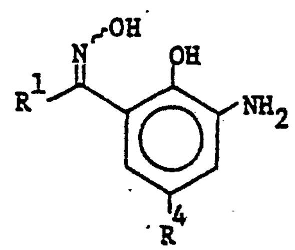

(IC)

[wherein $R^1$ and $R^4$ are as defined in claim 1],
subjecting to oxime forming a compound of the general formula (IB), and optionally converting any of the compounds of the formulae (IA), (IB), (IC) and (ID) to a pharmaceutically acceptable acid addition salt thereof.

44. A pharmaceutical composition which comprise, as active ingredient, at least one compound as claimed in any preceding claim or a pharmaceutically acceptable non-toxic sale thereof, together with a pharmaceutical carrier or coating.

CLAIMS for the Contracting State:-
AT

1. A method for the preparation of a 2-aminophenol derivative of the general formula (I) or a pharmaceutically acceptable acid addition salt thereof:

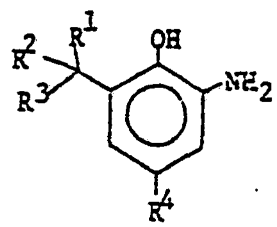

(I)

[wherein $R^1$ represents a straight or branched chain alkyl group containing from 1 to 6 carbon atom(s), a group represented by general formula:

$$-(CH_2)n-COOR^5 \qquad (II)$$

(wherein $R^5$ represents a hydrogen atom or a straight or branched chain alkyl group containing from 1 to 4 carbon atom(s) and n represents zero or an integer from 1 to 6) or a group represented by general formula:

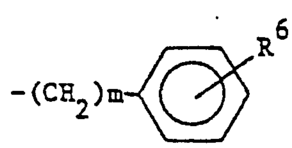

(III)

(wherein $R^6$ represents a hydrogen atom, a halogen atom, a straight or branched chain alkyl group containing from 1 to 4 carbon atom(s), a straight or branched chain alkoxyl group containing from 1 to 4 carbon atom(s), a carbamoyl group, an alkoxycarbonyl group containing from 2 to 6 carbon atoms, a carboxyl group, an amino group, a sulfamoyl group or a cyano group and m represents zero or an integer of 1 or 2.), $R^2$ represents a hydroxyl group, a straight or branched chain alkoxyl group containing from 1 to 4 carbon atom(s) or together with $R^3$ an oxo group or a hydroxyimino group, $R^3$ represents a hydrogen atom or together with $R^2$ sn oxo group or a hydroxyimino group, and $R^4$ represents a halogen atom, a straight or branched chain alkyl group containing from 1 to 6 carbon atom(s) or a phenylthio group, but not including a compound wherein $R^2$ and $R^3$ taken together represent an oxo group, $R^1$ represents a methyl group and $R^4$ represents a methyl group] which comprises selectively reducing the nitro group of a compound of the general formula:

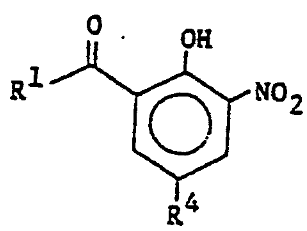

(IV)

[wherein $R^1$ and $R^4$ are as defined above], to an amino group to obtain a compound of the general formula:

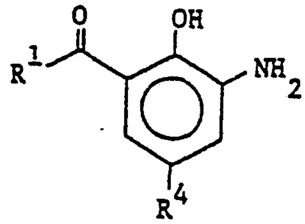

(IB)

[wherein $R^1$ and $R^4$ are as defined above] and, if desired to obtain a compound of the general formula:

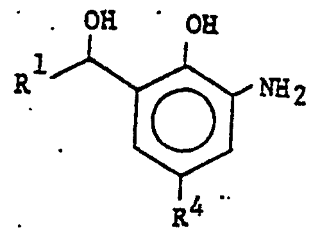

(IA)

[wherein $R^1$ and $R^4$ are as defined above], selectively reducing the oxo group of a compound of the general formula (IB) to a hydroxyl group; and further, if desired to obtain a compound of the general formula:

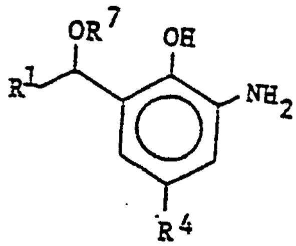

(ID)

[wherein $R^7$ represents a straight or branched chain alkyl group containing from 1 to 4 carbon atom(s) and $R^1$ and $R^4$ are as defined above],

converting the hydroxyl group of the α-position of a compound of the general formula (IA) to an alkoxyl group represented by $-OR^7$; or if desired to obtain a compound of the general formula:

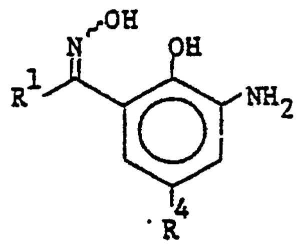

(IC)

[wherein $R^1$ and $R^4$ are as defined above], subjecting to oxine forming a compound of the general formula (IB), and optionally converting any of the compounds of the formulae (IA), (IB), (IC) and (ID) to a pharmaceutically acceptable acid addition salt thereof.

2. A method according to claim 1, wherein $R^2$ represents a hydroxyl group and $R^3$ represents a hydrogen atom and wherein a compound of the formula (IA) or a pharmaceutically acceptable acid addition salt thereof is obtained.

3. A method according to claim 1, wherein $R^2$ and $R^3$ taken together represents an oxo group and wherein a compound of the formula (IB) or a pharmaceutically acceptable acid addition salt thereof is obtained.

4. A method according to claim 1, wherein $R^2$ and $R^3$ taken together represent a hydroxyimino group and wherein a compound of the formula (IC) or a pharmaceutically acceptable acid addition salt thereof is obtained.

5. A method according to claim 1, wherein $R^2$ represents an alkoxyl group containing from 1 to 4 carbon atom(s) and $R^3$ represents a hydrogen atom, and wherein a compound of the formula (IC) or a pharmaceutically acceptable acid addition salt thereof is obtained.

6. A method according to claim 2 wherein $R^1$ represents the group represented by the general formula (III).

7. A method according to claim 3, wherein $R^1$ represents a group represented by the general formula (III).

8. A method according to claim 4, wherein $R^1$ represents a group represented by the general formula (III).

9. A method according to claim 5, wherein $R^1$ represents a group represented by the general formula (III).

10. A method according to claim 2, wherein $R^1$ represents an alkyl group containing from 1 to 6 carbon atom.

11. A method according to claim 3, wherein $R^1$ represents an alkyl group containing from 1 to 6 carbon atoms or a group represented by the general formula (II).

12. A method according to claim 6, wherein $R^6$ represents a holgen atom and $R^4$ represents a straight or branched chain alkyl group containing from 1 to 6 carbon atoms, or a halogen atom.

13. A method according to claim 12, wherein the product is 3-amino-5,4'-dichloro-2-hydroxybenzhydrol or its hydrochloride.

14. A method according to claim 12, wherein the product is 3-amino-4'-chloro-5-fluoro-2-hydroxybenzhydrol or its hydrochloride.

15. A method according to claim 12, wherein the product is 3-amino-5-t-butyl-4'-chloro-2-hydroxybenzhydrol or its hydrochloride.

16. A method according to claim 6, wherein $R^6$ represents a straight or branched chain alkoxyl group containing from 1 to 4 carbon atom(s) or an alkoxycarbonyl group containing from 2 to 6 carbon atoms, and $R^4$ represents a halogen atom or a phenylthio group.

17. A method according to claim 16, wherein the product is 3-amino-5-chloro-2-hydroxy-4'-methoxybenzhydrol or its hydrochloride.

18. A method according to claim 16, wherein the product is 3-amino-5-chloro-2-hydroxy-4'-methoxycarbonylbenzhydrol or its hydrochloride.

19. A method according to claim 16, wherein the product is 3-amino-2-hydroxy-4'-methoxy-5-phenylthiobenzhydrol or its hydrochloride.

20. A method according to claim 10, wherein $R^4$ represents a straight or branched chain alkyl group containing from 1 to 6 carbon atom(s).

21. A method according to claim 20, wherein the product is 2-amino-4-t-butyl-6-(1-hydroxyheptyl) phenol or its hydrochloride.

22. A method according to claim 7, wherein $R^6$ represents a hydrogen atom and $R^4$ represents a halgoen atom or a straight or branched chain alkyl group containing from 1 to 6 carbon atom(s).

23. A method according to claim 22, wherein the product is 3-amino-5-t-butyl-2-hydroxybenzophenone or its hydrochloride.

24. A method according to claim 22, wherein the product is 3-amino-5-chloro-2- hydroxybenzophenone or its hydrochloride.

25. A method according to claim 7, wherein $R^6$ represents a halogen atom and $R^4$ represents a halogen atom or a straight or branched chain alkyl group containing from 1 to 6 carbon atom(s).

26. A method according to claim 25, wherein the product is 3-amino-5,4'-dichloro-2-hydroxybenzophenone or its hydrochloride.

27. A method according to claim 25, wherein the product is 3-amino-4'-chloro-5-fluoro-2-hydroxybenzophenone or its hydrochloride.

28. A method according to claim 25, wherein the product is 3-amino-5-t-butyl-4'-chloro-2-hydroxybenzophenone or its hydrochloride.

29. A method according to claim 7, wherein $R^6$ represents a straight or branched chain alkoxyl group containing from 1 to 4 carbon atom(s) or an alkoxycarbonyl group containing from 2 to 6 carbon atoms and $R^4$ represents a halogen atom or a phenylthio group.

30. A method according to claim 29, wherein the product is 3-amino-5-chloro-2-hydroxy-4'-methoxybenzophenone or its hydrochloride.

31. A method according to claim 29, wherein the product is 3-amino-5-chloro-2-hydroxy-4'methoxycarbonylbenzophenone or its hydrochloride.

32. A method according to claim 29, wherein the product is 3-amino-2-hydroxy-4'-methoxy-5-phenylthiobenzophenone or its hydrochloride.

33. A method according to claim 11, wherein $R^1$ represents a straight or branched chain alkyl group containing from 1 to 6 carbon atom(s) and $R^4$ represents a halogen atom or a straight or branched chain alkyl group containing from 1 to 6 carbon atom(s).

34. A method according to claim 33, wherein the product is 2-amino-4-chloro-6-heptanoylphenol or its hydrochloride.

35. A method according to claim 33, wherein the product is 2-amino-4-t-butyl-6-heptanoylphenol or its hydrochloride.

36. A method according to claim 33, wherein the product is 2-amino-4-t-butyl-6-propionylphenol or its hydrochloride.

37. A method according to claim 11, wherein $R^1$ represents the group represented by the general formula (II) and $R^4$ represents a straight or branched chain alkyl group containing from 1 to 6 carbon atom(s).

38. A method according to claim 37, wherein the product is 2-amino-4-t-butyl-6-(4-ethoxycarbonylbutyryl)phenol or its hydrochoride.

39. A method according to claim 8, wherein $R^6$ represents a halogen atom and $R^4$ represents a halogen atom.

40. A method according to claim 39, wherein the product is 3-amino-4'-chloro-5-fluoro-2-hydroxybenzophenoneoxime or its hydrochloride.

41. A method according to claim 9, wherein $R^6$ represents a straight or branched chain alkoxyl group containing from 1 to 4 carbon atom(s) and $R^4$ represents a halogen atom.

42. A method according to claim 41, wherein the product is 2-amino-4-chloro-6-(α-ethoxy-4-methoxybenzyl)phenol or its hydrochloride.

0065874

European Patent Office

# EUROPEAN SEARCH REPORT

Application number

EP 82 30 2598

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | FR-A-2 407 207 (MAY & BAKER) *Pages 12,13,23,25-26,35-36-37* & GB - A - 2 006 782 | 1,3 | C 07 C 97/10<br>C 07 C 91/44<br>C 07 C 93/14<br>C 07 C 101/72<br>C 07 C 101/78<br>C 07 C 149/42<br>C 07 C 131/00<br>A 61 K 31/135<br>A 61 K 31/15<br>A 61 K 31/195<br>A 61 K 31/215 |
| A | GB-A-2 026 480 (ONO PHARMACEUTICAL) *Claims* | 1,44 | |
| | | | TECHNICAL FIELDS SEARCHED (Int. Cl. 3) |
| | | | C 07 C 91/00<br>C 07 C 93/00<br>C 07 C 97/00<br>C 07 C 101/00<br>C 07 C 131/00<br>C 07 C 149/00<br>A 61 K 31/00 |

The present search report has been drawn up for all claims

| Place of search | Date of completion of the search | Examiner |
|---|---|---|
| THE HAGUE | 12-08-1982 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82